# EUROPEAN PATENT SPECIFICATION

(11) **EP 3 936 055 B1**
(45) Date of publication and mention of the grant of the patent: **01.03.2023**
(21) Application number: 20771068.2
(22) Date of filing: 21.02.2020
(51) Int. Cl.: A61B 8/14

(54) **ULTRASONIC DIAGNOSTIC DEVICE AND METHOD FOR CONTROLLING ULTRASONIC DIAGNOSTIC DEVICE**
ULTRASCHALLDIAGNOSEVORRICHTUNG UND VERFAHREN ZUR STEUERUNG DER ULTRASCHALLDIAGNOSEVORRICHTUNG
DISPOSITIF DE DIAGNOSTIC ULTRASONORE ET PROCÉDÉ DE COMMANDE DE DISPOSITIF DE DIAGNOSTIC ULTRASONORE

(30) Priority: 08.03.2019 JP 2019042493
(43) Date of publication of application: 12.01.2022
(73) Proprietor: FUJIFILM Corporation, Tokyo 106-8620 (JP)
(72) Inventor: EBATA Tetsurou, Ashigarakami-gun, Kanagawa 258-8538 (JP); INOUE Tomoki, Ashigarakami-gun, Kanagawa 258-8538 (JP)
(74) Representative: Kudlek, Franz Thomas
(86) International application number: PCT/JP2020/006954
(87) International publication number: WO 2020/184144

(56) References cited:
- EP-B1- 2 010 060
- JP-A- 2016 140 589
- US-A1- 2017 296 148

## Description

### BACKGROUND OF THE INVENTION

### 1. Field of the Invention

The present invention relates to an ultrasound diagnostic apparatus and a method of controlling an ultrasound diagnostic apparatus, and in particular, to an ultrasound diagnostic apparatus and a method of controlling an ultrasound diagnostic apparatus that measure a urine volume in a bladder of a subject.

### 2. Description of the Related Art

Hitherto, in a medical field, an ultrasound diagnostic apparatus using an ultrasound image has come into practical use. In general, this kind of ultrasound diagnostic apparatus has an ultrasound probe that incorporates a transducer array, and an apparatus body connected to the ultrasound probe. The ultrasound probe transmits ultrasonic waves toward a subject and receives ultrasound echoes from the subject, and the apparatus body electrically processes reception signals to generate an ultrasound image.

A bladder of the subject is observed using such an ultrasound diagnostic apparatus, and a urine volume in the observed bladder is measured. In general, the urine volume in the bladder of the subject is substantially equal to the volume of the bladder of the subject, and thus, the volume of the bladder of the subject is measured as the urine volume. Although the volume of the bladder of the subject can be calculated, for example, using the maximum diameter in a longitudinal direction, the maximum diameter in the lateral direction, and the maximum diameter in a depth direction of the bladder while regarding the bladder as an ellipsoid, normally, to obtain the maximum diameters of the bladder in the longitudinal direction, the lateral direction, and the depth direction, the user needs to move the ultrasound probe to observe a tomographic image of the bladder in which a diameter of the bladder in the longitudinal direction is maximized and a tomographic image of the bladder in which a diameter of the bladder in the lateral direction is maximized, and to manually measure the diameter of the bladder.

Accordingly, to save labor of the user to manually measure the diameter of the bladder on the tomographic image, as disclosed in JP2017-109074A, an ultrasound diagnostic apparatus that automatically extracts a bladder region in an ultrasound image in acquired ultrasound images of a plurality of frames, measures a diameter of the extracted bladder region, and measures the volume of a bladder of a subject based on the measured diameter of the bladder region.

### SUMMARY OF THE INVENTION

However, in the ultrasound diagnostic apparatus of JP2017-109074A, for example, in a case where the bladder region is incorrectly extracted for the reason that an ultrasound image where the bladder of the subject is not clearly shown is included in the acquired ultrasound images of the plurality of frames, or the like, there is a problem regarding measurement accuracy of the urine volume, such as a case where an incorrect value is calculated on the volume of the bladder, that is, the urine volume.

The invention has been accomplished to solve the problem in the related art, and an object of the invention is to provide an ultrasound diagnostic apparatus and a method of controlling an ultrasound diagnostic apparatus capable of measuring a urine volume in a bladder of a subject with excellent accuracy.

To achieve the above-described object, an ultrasound diagnostic apparatus according to an aspect of the invention comprises an ultrasound probe that is brought into contact with a subject and scans the subject with an ultrasound beam, an image acquisition unit that acquires ultrasound images of a plurality of frames corresponding to a plurality of different tomographic planes in the subject using the ultrasound probe, an image memory that keeps the ultrasound images of the plurality of frames acquired by the image acquisition unit, a bladder extraction unit that extracts a bladder region from each of the ultrasound images of the plurality of frames, a feature value calculation unit that calculates a feature value regarding the bladder region extracted by the bladder extraction unit in each of the ultrasound images of the plurality of frames, and a scanning success/failure determination unit that analyzes change in the feature value calculated by the feature value calculation unit between frames continuous in time series and determines whether or not scanning of a bladder of the subject with the ultrasound beam is successful based on an analysis result.

It is preferable that the ultrasound images of the plurality of frames are ultrasound images that are acquired by the image acquisition unit while changing a tilt angle of the ultrasound probe over a given angle range with respect to the subject while a contact position of the ultrasound probe with the subject is fixed.

It is preferable that the ultrasound diagnostic apparatus further comprises a maximum diameter measurement unit that measures a maximum diameter of the bladder region using the ultrasound images of the plurality of frames in a case where the scanning success/failure determination unit determines that the scanning with the ultrasound beam is successful.

The ultrasound images of the plurality of frames may be ultrasound images that are acquired by the image acquisition unit in a case where a plurality of times of scanning of the bladder of the subject with the ultrasound beam are performed.

In this case, the scanning success/failure determination unit may determine whether or not each of the plurality of times of scanning with the ultrasound beam is successful.

It is preferable that the ultrasound diagnostic apparatus further comprises a maximum diameter measurement unit that measures a maximum diameter of the bladder region using only ultrasound images of a plurality of frames acquired by scanning with the ultrasound beam determined to be successful by the scanning success/failure determination unit among the plurality of times of scanning with the ultrasound beam.

It is preferable that the feature value is an area or a diameter of the bladder region extracted by the bladder extraction unit in the ultrasound image.

In this case, the scanning success/failure determination unit may determine whether or not the scanning of the bladder of the subject with the ultrasound beam is successful by analyzing continuity of change in the feature value between frames continuous in time series.

More specifically, the scanning success/failure determination unit may determine that the scanning with the ultrasound beam is successful in a case where a difference value of the feature values between any frames continuous in time series in the ultrasound images of the plurality of frames is smaller than a given threshold value, and may determine that the scanning with the ultrasound beam fails in a case where a difference value of the feature values in at least one frame continuous in time series in the ultrasound images of the plurality of frames is equal to or greater than the threshold value.

Alternatively, the ultrasound images of the plurality of frames may be ultrasound images that are acquired by the image acquisition unit while changing the tilt angle of the ultrasound probe at a constant speed while the contact position of the ultrasound probe with the subject is fixed, and in this case, the scanning success/failure determination unit may determine that the scanning with the ultrasound beam is successful in a case where a difference value of the feature values between any frames continuous in time series in the ultrasound images of the plurality of frames is smaller than a given threshold value, and symmetry of a distribution of the feature values with respect to a time axis is acknowledged, and may determine that the scanning with the ultrasound beam fails in a case where a difference value of the feature values in at least one frame continuous in time series in the ultrasound images of the plurality of frames is equal to or greater than the threshold value or in a case where the symmetry of the distribution of the feature values with respect to the time axis is not acknowledged.

For example, the scanning success/failure determination unit may determine that the symmetry of the distribution of the feature values with respect to the time axis is acknowledged in a case where a position where the feature value is maximized is positioned in a middle point of a pair of positions where the feature value is minimized, on the time axis, and may determine that the symmetry of the distribution of the feature values with respect to the time axis is not acknowledged in a case where the position where the feature value is maximized deviates from the middle points of the pair of positions where the feature value is minimized, on the time axis.

The ultrasound diagnostic apparatus may further comprise a tilt angle sensor that measures the tilt angle of the ultrasound probe, and the scanning success/failure determination unit may determine that the scanning with the ultrasound beam is successful in a case where a difference value of the feature values between any frames continuous in time series in the ultrasound images of the plurality of frames is smaller than a given threshold value, and a position where the feature value indicates an extreme value is acknowledged to coincide with a position where the tilt angle measured by the tilt angle sensor indicates an extreme value, on a time axis, and may determine that the scanning with the ultrasound beam fails in a case where a difference value of the feature values in at least one frame continuous in time series is equal to or greater than the threshold value or the position where the feature value with respect to the time axis indicates the extreme value is not acknowledged to coincide with the position where the tilt angle measured by the tilt angle sensor indicates the extreme value.

In this case, the ultrasound diagnostic apparatus may further comprise a target distance estimation unit that estimates a distance between a center of the bladder of the subject and the contact position of the ultrasound probe with the subject in a direction along a body surface of the subject based on an ultrasound image of a frame representing a tomographic plane passing the center of the bladder of the subject among the ultrasound images of the plurality of frames acquired by the image acquisition unit and the tilt angle of the ultrasound probe measured by the tilt angle sensor, and a probe movement guidance unit that guides a user to position the ultrasound probe directly above the center of the bladder of the subject by moving the ultrasound probe along the body surface of the subject by the distance estimated by the target distance estimation unit.

The feature value may be a position of the bladder region extracted by the bladder extraction unit in the ultrasound image.

For example, the feature value may be a position of a center of gravity of the bladder region extracted by the bladder extraction unit in the ultrasound image, and in this case, the scanning success/failure determination unit may determine that the scanning with the ultrasound beam is successful in a case where a distance between the centers of gravity of the bladder regions in frames continuous in time series is smaller than a given threshold value, and may determine that the scanning with the ultrasound beam fails in a case where the distance between the centers of gravity of the bladder regions in the frames continuous in time series is equal to or greater than the given threshold value.

The scanning success/failure determination unit may determine that the scanning with the ultrasound beam is successful in a case where a ratio of an area of a region where the bladder regions overlap each other to an area of a region occupied by at least one of the bladder regions in the frames continuous in time series is equal to or greater than a given threshold value, and may determine that the scanning of the ultrasound beam fails in a case where the ratio of the area of the region where the bladder regions overlap each other to the area of the region occupied by at least one of the bladder regions in the frames continuous in time series is smaller than the given threshold value.

The ultrasound diagnostic apparatus may further comprise a notification unit that, in a case where the scanning success/failure determination unit determines that the scanning of the ultrasound beam fails, notifies a user that the scanning of the ultrasound beam fails.

It is preferable that the image acquisition unit acquires a first group of ultrasound images of a plurality of frames corresponding to the plurality of tomographic planes of the bladder of the subject along a lateral direction and a second group of ultrasound images of a plurality of frames corresponding to the plurality of tomographic planes of the bladder of the subject along a longitudinal direction, the bladder extraction unit extracts the bladder region from each of the first group of the ultrasound images of the plurality of frames and the second group of the ultrasound images of the plurality of frames, the feature value calculation unit calculates the feature value from each of the first group of the ultrasound images of the plurality of frames and the second group of the ultrasound images of the plurality of frames, and the scanning success/failure determination unit determines whether or not the scanning of the bladder of the subject with the ultrasound beam is successful for each of the first group of the ultrasound images of the plurality of frames and the second group of the ultrasound images of the plurality of frames.

In this case, it is preferable that the ultrasound diagnostic apparatus further comprises a bladder volume calculation unit that calculates a volume of the bladder based on a maximum diameter of the bladder region measured from the first group of the ultrasound images of the plurality of frames in the lateral direction, a maximum diameter of the bladder region measured from the second group of the ultrasound images of the plurality of frames in the longitudinal direction, and a maximum diameter of the bladder region measured from the first group of the ultrasound images of the plurality of frames or the second group of the ultrasound images of the plurality of frames in a depth direction.

A method of controlling an ultrasound diagnostic apparatus according to another aspect of the invention comprises acquiring ultrasound images of a plurality of frames corresponding to a plurality of different tomographic planes in a subject using an ultrasound probe that is brought into contact with the subject and scans the subject with an ultrasound beam, keeping the acquired ultrasound images of the plurality of frames, extracting a bladder region from each of the ultrasound images of the plurality of frames, calculating a feature value regarding the bladder region extracted in each of the ultrasound images of the plurality of frames, and analyzing change in the calculated feature value between frames continuous in time series and determining whether or not scanning of a bladder of the subject with the ultrasound beam is successful based on an analysis result.

The ultrasound diagnostic apparatus according to the aspect of the invention comprises the bladder extraction unit that extracts the bladder region from each of the ultrasound images of the plurality of frames, the feature value calculation unit that calculates the feature value regarding the bladder region extracted by the bladder extraction unit in each of the ultrasound images of the plurality of frames, and the scanning success/failure determination unit that analyzes change in feature value calculated by the feature value calculation unit between the frames continuous in time series and determines whether or not the scanning of the bladder of the subject with the ultrasound beam is successful based on the analysis result. Therefore, it is possible to measure the urine volume in the bladder of the subject with excellent accuracy.

### BRIEF DESCRIPTION OF THE DRAWINGS

Fig. 1 is a block diagram showing the configuration of an ultrasound diagnostic apparatus according to Embodiment 1 of the invention.
Fig. 2 is a block diagram showing the configuration of a reception unit in Embodiment 1 of the invention.
Fig. 3 is a block diagram showing the configuration of an image generation unit in Embodiment 1 of the invention.
Fig. 4 is a diagram schematically showing an ultrasound probe in contact with a subject and a bladder of the subject positioned directly below the ultrasound probe in Embodiment 1 of the invention.
Fig. 5 is a schematic view showing a scene in which the ultrasound probe is tilted in Embodiment 1 of the invention.
Fig. 6 is an example of a continuous graph representing a relationship between a time at which an ultrasound image of each frame is acquired and an area of a bladder region included in the ultrasound image Embodiment 1 of the invention.
Fig. 7 is an example of a discontinuous graph representing a relationship between a time at which an ultrasound image of each frame is acquired and an area of a bladder region included in the ultrasound image in Embodiment 1 of the invention.
Fig. 8 is another example of a discontinuous graph showing a relationship between a time at which an ultrasound image of each frame is acquired and an area of a bladder region included in the ultrasound image in Embodiment 1 of the invention.
Fig. 9 is a diagram showing an example of an ellipsoid.
Fig. 10 is a diagram showing an example of a contact position of the ultrasound probe with the subject in Embodiment 1 of the invention.
Fig. 11 is a diagram schematically showing an ultrasound image representing a tomographic plane where a diameter of a bladder of the subject in a lateral direction is maximized.
Fig. 12 is a diagram schematically showing an ultrasound image representing a tomographic plane where a diameter of the bladder of the subject in a longitudinal direction is maximized.
Fig. 13 is a flowchart showing the operation of the ultrasound diagnostic apparatus in Embodiment 1 of the invention.
Fig. 14 is a diagram showing an example where information indicating that scanning of a bladder of a subject with an ultrasound beam fails is displayed on a display unit.
Fig. 15 is a diagram showing an example where information for giving a command to image a longitudinal tomographic plane of the bladder of the subject is displayed on the display unit.
Fig. 16 is an example of a discontinuous graph representing a relationship representing a time at which an ultrasound image of each frame is acquired and an area of a bladder region included in the ultrasound image in a modification example of Embodiment 1 of the invention.
Fig. 17 is a diagram schematically showing a distance between centers of gravity of bladder regions in frames continuous in time series.
Fig. 18 is an example of a graph representing a relationship between a time at which an ultrasound image of each frame is acquired and a distance between centers of gravity of bladder regions in frames continuous in time series in a modification example of Embodiment 1.
Fig. 19 is a diagram schematically showing a region where bladder regions in frames continuous in time series overlap each other and a region occupied by at least one of the bladder regions.
Fig. 20 is an example of a continuous graph representing a relationship between a time at which an ultrasound image of each frame is acquired and a ratio of an area of a region where bladder regions in frames continuous in time series overlap each other to an area of a region occupied by at least one of the bladder regions in a modification example of Embodiment 1.
Fig. 21 is an example of a discontinuous graph representing a relationship between a time at which an ultrasound image of each frame is acquired and a ratio of an area of a region where bladder regions in frames continuous in time series overlap each other to an area of a region occupied by at least one of the bladder regions in a modification example of Embodiment 1.
Fig. 22 is an example of a continuous graph representing a relationship between a time at which an ultrasound image is acquired and an area of a bladder region included in the ultrasound image in a case where a plurality of times of scanning of a bladder of a subject with an ultrasound beam are performed in Embodiment 2 of the invention.
Fig. 23 is an example of a discontinuous graph representing a relationship between a time at which an ultrasound image is acquired and an area of a bladder region included in the ultrasound image in a case where a plurality of times of scanning of a bladder of a subject with an ultrasound beam is performed in Embodiment 2 of the invention.
Fig. 24 is an example of a symmetric graph representing a relationship between a time at which an ultrasound image of each frame is acquired and an area of a bladder region included in the ultrasound image in Embodiment 3 of the invention.
Fig. 25 is a diagram schematically showing a scene in which the ultrasound probe is brought into contact with the subject at a position off from directly above a center of a bladder of the subject in Embodiment 3 of the invention.
Fig. 26 is an example of an asymmetric graph representing a relationship between a time at which an ultrasound image of each frame is acquired and an area of a bladder region included in the ultrasound image in Embodiment 3 of the invention.
Fig. 27 is a block diagram showing the configuration of an ultrasound diagnostic apparatus according to Embodiment 4 of the invention.
Fig. 28 is a graph showing that a position on a time axis where an area of a bladder region indicates an extreme value coincides with a position on the time axis where a tilt angle of the ultrasound probe indicates an extreme value in Embodiment 4 of the invention.
Fig. 29 is a graph showing that a position on a time axis where an area of a bladder region indicates an extreme value is different from a position on the time axis where a tilt angle of the ultrasound probe indicates an extreme value in Embodiment 4 of the invention.
Fig. 30 is a schematic view showing a state in which a contact position of the ultrasound probe deviates from directly above the center of the bladder of the subject.
Fig. 31 is a diagram showing an example where information indicating guidance of movement of the ultrasound probe is displayed on a display unit.
Fig. 32 is a diagram showing the configuration of an ultrasound diagnostic apparatus according to Embodiment 5 of the invention.

### DESCRIPTION OF THE PREFERRED EMBODIMENTS

Hereinafter, embodiments of the invention will be described referring to the accompanying drawings.

The description of constituent elements described below is provided based on a representative embodiment of the invention, but the invention is not limited to such an embodiment.

### Embodiment 1

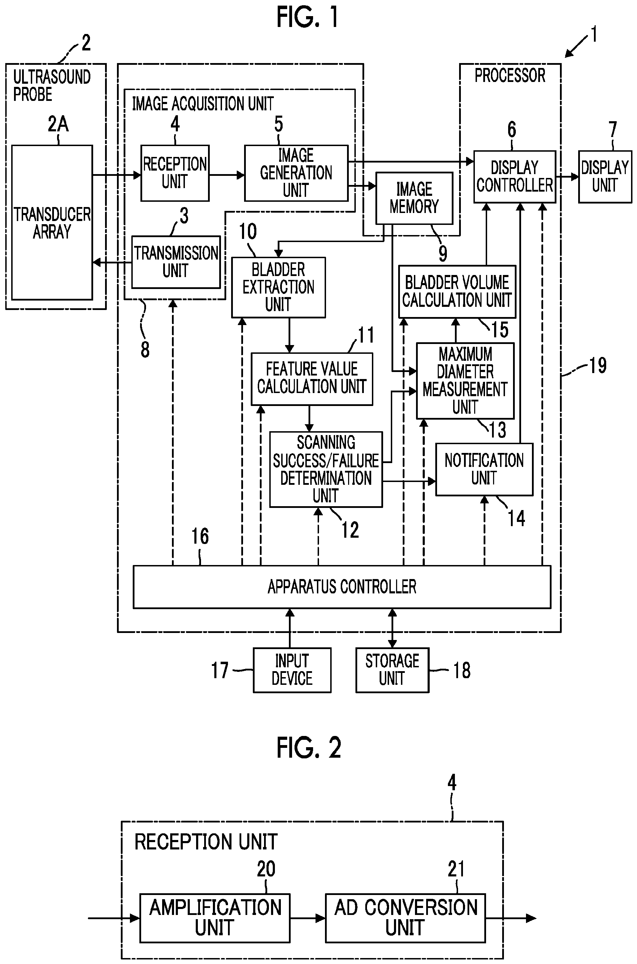

Fig. 1 shows an ultrasound diagnostic apparatus 1 according to Embodiment 1 of the invention. The ultrasound diagnostic apparatus 1 comprises an ultrasound probe 2 that incorporates a transducer array 2A, and a transmission unit 3 and a reception unit 4 are connected to the transducer array 2A. An image generation unit 5, a display controller 6, and a display unit 7 are sequentially connected to the reception unit 4. Here, the transmission unit 3, the reception unit 4, and the image generation unit 5 configure an image acquisition unit 8. An image memory 9 is connected to the image generation unit 5, and a bladder extraction unit 10, a feature value calculation unit 11, and a scanning success/failure determination unit 12 are sequentially connected to the image memory 9. A maximum diameter measurement unit 13 and a notification unit 14 are connected to the scanning success/failure determination unit 12. The image memory 9 and a bladder volume calculation unit 15 are connected to the maximum diameter measurement unit 13, and a display controller 6 is connected to the bladder volume calculation unit 15. The display controller 6 is connected to the notification unit 14.

An apparatus controller 16 is connected to the display controller 6, the image acquisition unit 8, the bladder extraction unit 10, the feature value calculation unit 11, the scanning success/failure determination unit 12, the maximum diameter measurement unit 13, the notification unit 14, and the bladder volume calculation unit 15, and an input device 17 and a storage unit 18 are connected to the apparatus controller 16. Here, the apparatus controller 16 and the storage unit 18 are connected to be transferrable information in two directions.

The display controller 6, the image acquisition unit 8, the bladder extraction unit 10, the feature value calculation unit 11, the scanning success/failure determination unit 12, the maximum diameter measurement unit 13, the notification unit 14, the bladder volume calculation unit 15, and the apparatus controller 16 configure a processor 19.

The transducer array 2A of the ultrasound probe 2 shown in Fig. 1 has a plurality of transducers arranged in a one-dimensional or two-dimensional manner. The transducers transmit ultrasonic waves in compliance with drive signals supplied from the transmission unit 3, receive ultrasound echoes from a subject, and output reception signals. Each transducer is configured by forming electrodes at both ends of a piezoelectric body made of, for example, piezoelectric ceramic represented by lead zirconatetitanate (PZT), a polymer piezoelectric element represented by poly vinylidene di fluoride (PVDF), piezoelectric single crystal represented by lead magnesium niobate-lead titanate (PMN-PT), or the like.

The transmission unit 3 of the image acquisition unit 8 includes, for example, a plurality of pulse generators, and adjusts a delay amount of each drive signal based on a transmission delay pattern selected in response to a control signal from the apparatus controller 16 such that the ultrasonic waves transmitted from a plurality of transducers of the transducer array 2A form an ultrasound beam, and supplies the drive signals to a plurality of transducers. In this way, in a case where a pulsed or continuous-wave voltage is applied to the electrodes of each of a plurality of transducers of the transducer array 2A, the piezoelectric body expands and contracts to generate a pulsed or continuous-wave ultrasonic wave from each of the transducers. An ultrasound beam is formed from a combined wave of the ultrasonic waves.

The transmitted ultrasound beam is reflected by, for example, a target, such as a part of the subject, and propagates toward the transducer array 2A of the ultrasound probe 2. The ultrasound echo propagating toward the transducer array 2A is received by each transducer configuring the transducer array 2A. In this case, each transducer configuring the transducer array 2A expands and contracts with reception of the propagating ultrasound echo to generate an electrical signal, and outputs the electrical signal to the reception unit 4.

The reception unit 4 of the image acquisition unit 8 executes processing of the signals output from the transducer array 2A in compliance with a control signal from the apparatus controller 16. As shown in Fig. 2, the reception unit 4 has a configuration in which an amplification unit 20 and an analog-digital (AD) conversion unit 21 are connected in series. The amplification unit 20 amplifies the signal input from each transducer configuring the transducer array 2A and transmits the amplified signal to the AD conversion unit 21. The AD conversion unit 21 converts the signal transmitted from the amplification unit 20 into a digitized reception signal and sends the converted data to the image generation unit 5 of the image acquisition unit 8.

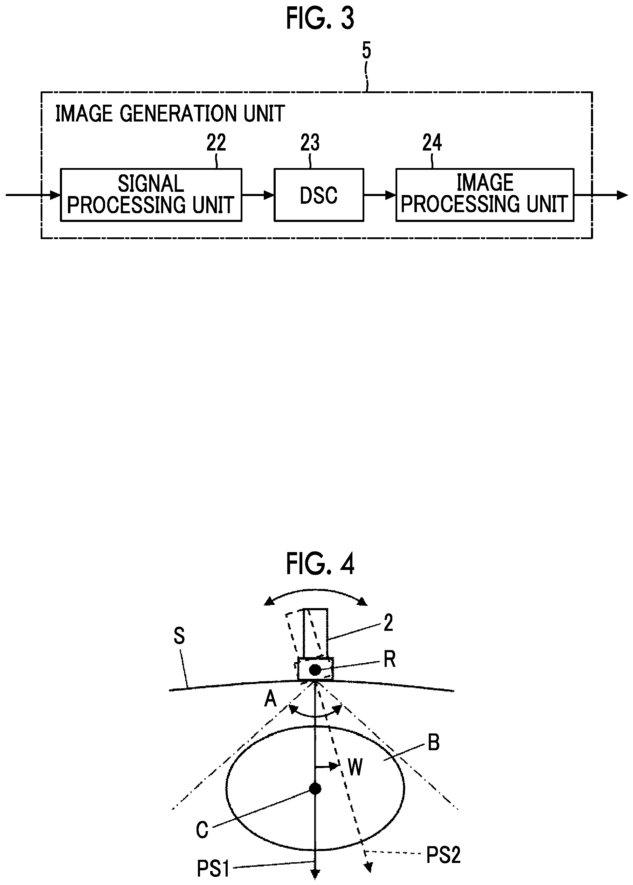

As shown in Fig. 3, the image generation unit 5 of the image acquisition unit 8 has a configuration in which a signal processing unit 22, a digital scan converter (DSC) 23, and an image processing unit 24 are sequentially connected in series. The signal processing unit 22 executes reception focus processing of giving each delay to each piece of data of the reception signal based on a reception delay pattern in response to a control signal from the apparatus controller 16 and performs addition (phasing addition). With the reception focus processing, a sound ray signal in which the focus of the ultrasound echo is narrowed to one scanning line is generated. The signal processing unit 22 performs correction of attenuation due to a propagation distance on the generated sound ray signal corresponding to a depth of a position where the ultrasonic wave is reflected, and then, executes envelope detection processing to generate a B mode image signal representing a tissue in the subject. The B mode image signal generated in this way is output to the DSC 23.

The DSC 23 of the image generation unit 5 raster-converts the B mode image signal into an image signal compliant with a normal television signal scanning system to generate an ultrasound image. The image processing unit 24 of the image generation unit 5 executes various kinds of image processing, such as brightness correction, gradation correction, sharpness correction, and color correction, on the ultrasound image obtained in the DSC 23, and then, outputs the ultrasound image to the display controller 6 and the image memory 9.

The image memory 9 sequentially keeps the ultrasound images obtained by the image acquisition unit 8. As the image memory 9, a recording medium, such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), or a universal serial bus memory (USB memory), a server, or the like can be used.

The bladder extraction unit 10 extracts a bladder region from each of ultrasound images of a plurality of frames generated by the image generation unit 5 of the image acquisition unit 8. The bladder extraction unit 10 can use, for example, a method using deep learning described in Krizhevsk et al.: ImageNet Classification with Deep Convolutional Neural Networks, Advances in Neural Information Processing Systems 25, pp. 1106-1114 (2012). The bladder extraction unit 10 can use known methods, such as graph-cut (Y Boykov and V. Kolmogorov, "An experimental comparison of min-cut/max-flow algorithm for energy minimization in vision", IEEE Transactions on Pattern Analysis and Machine Intelligence, 26, 9, pp. 1123-1137, 2004.), Snakes (A. W. Michael Kass and D. Terzopoulos: "Snakes: Active contour models", Int. J. Computer Vision, 1, 4, pp. 321-331, 1988.), and LevelSets (M. Sussman, P. Smereka and S. Osher: "A level set approach for computing solutions to incompressible two-phase flow", J. Comput. Phys, 114, 1, pp. 146-159, 1994), as other methods to extract the bladder region.

The feature value calculation unit 11 calculates a feature value regarding the extracted bladder region in each of the ultrasound images of the plurality of frames from which the bladder region is extracted by the bladder extraction unit 10. The feature value calculation unit 11 can calculate, for example, an area, a diameter, a position, or the like of the extracted bladder region as a feature value.

The scanning success/failure determination unit 12 analyzes change in feature value calculated by the feature value calculation unit 11 between frames continuous in time series and determines whether or not scanning of a bladder of the subject with an ultrasound beam is successful based on an analysis result. For example, the scanning success/failure determination unit 12 can determine whether or not the scanning of the bladder of the subject with the ultrasound beam is successful by analyzing continuity of change in feature value between frames continuous in time series. More specifically, for example, the scanning success/failure determination unit 12 determines that the scanning of the bladder of the subject with the ultrasound beam is successful in a case where a difference value of the feature values between any frames continuous in time series is smaller than a given threshold value, and determines that the scanning with the ultrasound beam fails in a case where a difference value of the feature values in at least one frame continuous in time series is equal to or greater than the threshold value.

Here, for example, as shown in Fig. 4, as the ultrasound probe 2 is positioned directly above a bladder B of a subject S and the ultrasound probe 2 is rotated over a given angle range A around a rotation axis R parallel to an arrangement direction of the transducer array 2A while a contact position of the ultrasound probe 2 with the subject S is fixed, that is, the ultrasound probe 2 is rotated using a so-called swing method, ultrasound images of a plurality of frames can be acquired by the image acquisition unit 8 while changing a tilt angle W of the ultrasound probe 2.

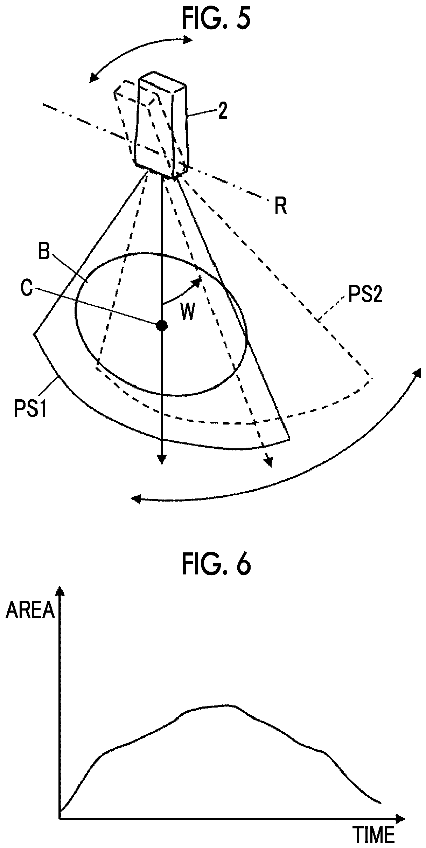

Here, the arrangement direction of the transducer array 2A and an extension direction of the rotation axis R are perpendicular to the paper surface in Fig. 4. As the ultrasound probe 2 rotates around the rotation axis R, as shown in Fig. 5, a scanning plane PS1 extending from the ultrasound probe 2 into the subject S also rotates around the rotation axis R.

The tilt angle W of the ultrasound probe 2 represents an angle at which the ultrasound probe 2 is tilted from a state in which a normal direction of the transducer array 2A is directed in a direction perpendicular to a body surface of the subject S at the center of the transducer array 2A of the ultrasound probe 2. That is, it is assumed that the tilt angle W indicates 0 degrees in the ultrasound probe 2 in a state in which the direction normal to the transducer array 2A is directed in the direction perpendicular to the body surface of the subject S, and has a greater value as the ultrasound probe 2 is further tilted from the state. In an example shown in Figs. 4 and 5, the tilt angle W is represented as a rotation angle between the scanning plane PS1 directed in the direction perpendicular to the body surface of the subject S and a scanning plane PS2 in a state in which the ultrasound probe 2 is tilted.

The given angle range A is an angle range such that the scanning plane extending from the transducer array 2A of the ultrasound probe 2 into the subject S passes through at least a center C of the bladder B of the subject S, and more preferably, a range of the tilt angle W of the ultrasound probe 2 such that the scanning plane extending from the transducer array 2A of the ultrasound probe 2 into the subject S passes through the whole of the bladder B of the subject S.

In a case where the tilt angle W of the ultrasound probe 2 changes at a substantially constant speed, and a bladder region is clearly visualized in all the ultrasound images of the plurality of frames, for example, as shown in Fig. 6, the feature values calculated by the feature value calculation unit 11 in the ultrasound images of the plurality of frames continuously changes in compliance with change in time. In an example shown in Fig. 6, the area of the bladder region in the ultrasound image is calculated as the feature value. In this way, in a case where change in feature value is continuous with respect to a time axis, the difference value of the feature values between any frames continuous in time series is smaller than the given threshold value. Thus, the scanning success/failure determination unit 12 determines that the scanning of the bladder B of the subject S with the ultrasound beam is successful.

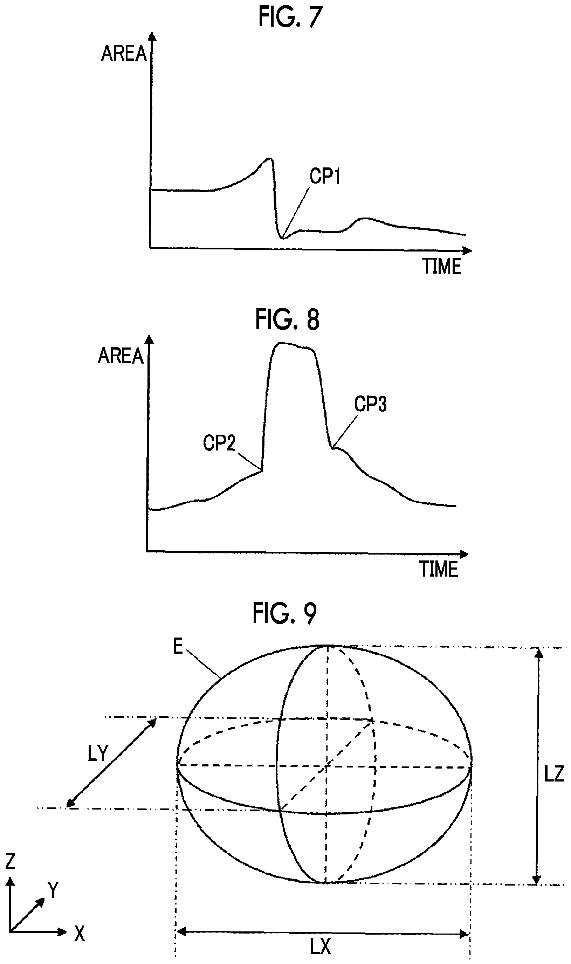

In a case where the tilt angle W of the ultrasound probe 2 changes extremely, in a case where the bladder region visualized in the ultrasound image is unclear, or the like, for example, as shown in Figs. 7 and 8, the feature values calculated by the feature value calculation unit 11 in the ultrasound images of the plurality of frames have a portion where a change amount is discontinuous in time series. In an example shown in Fig. 7, the area of the bladder region in the ultrasound image is calculated as the feature value, and includes a change point CP1 where the feature value changes extremely between frames continuous in time series. A change amount of the area of the bladder region at the change point CP1 is discontinuous. A difference between the area at the change point CP1 and the area of each bladder region in ultrasound images of frames preceding and following a frame corresponding to the change point CP1 is equal to or greater than the given threshold value. Thus, the scanning success/failure determination unit 12 determines that the scanning of the bladder B of the subject S with the ultrasound beam fails.

In an example shown in Fig. 8, the area of the bladder region in the ultrasound image is calculated as the feature value, and the calculated area of the bladder region includes change points CP2 and CP3 where the feature value changes extremely between frames continuous in time series. In this case, both a difference between the area at the change point CP2 and the area of two bladder regions in an ultrasound image of a frame following a frame corresponding to the change point CP2 and a difference between the area at the change point CP3 and the area of two bladder regions in an ultrasound image of a frame preceding a frame corresponding to the change point CP3 are equal to or greater than the given threshold value. Thus, the scanning success/failure determination unit 12 determines that the scanning of the bladder B of the subject S with the ultrasound beam fails.

In a case where the scanning success/failure determination unit 12 determines that the scanning of the bladder B of the subject S with the ultrasound beam is successful, the maximum diameter measurement unit 13 measures a diameter of the bladder B of the subject S that is used by the bladder volume calculation unit 15 to calculate the volume of the bladder B of the subject S. In general, since the bladder B has a substantially ellipsoid shape, the volume of the bladder B is calculated as the volume of the ellipsoid. As shown in Fig. 9, an ellipsoid E has a shape symmetric with respect to an XY plane, a YZ plane, and an XZ plane, and in a case where the maximum diameter of the ellipsoid E in the X direction is LX, the maximum diameter of the ellipsoid E in the Y direction is LY, the maximum diameter of the ellipsoid E in the Z direction is LZ, and a circular constant is π, the volume of the ellipsoid E can be calculated by (LX × LY × LZ)/(6π).

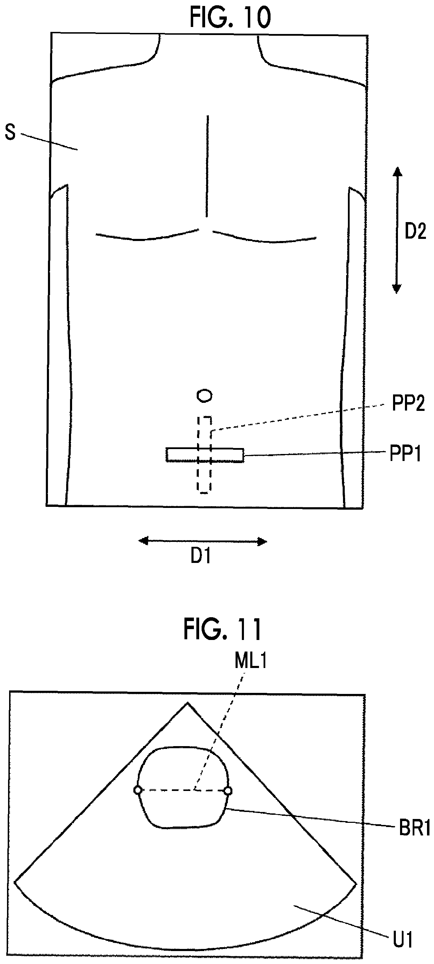

Accordingly, in a case of calculating the volume of the bladder B of the subject S, for example, as shown in Fig. 10, each of three directions of a lateral direction D1 in a case of facing the subject S from the front, a longitudinal direction D2 along a height direction of the subject S, and a depth direction (not shown) perpendicular to both the lateral direction D1 and the longitudinal direction D2 is regarded any of the X direction, the Y direction, or the Z direction in the ellipsoid E, and the ultrasound probe 2 is disposed at each of a first contact position PP1 for observing a tomographic plane of the bladder B along the lateral direction D1 of the subject S and a second contact position PP2 for observing a tomographic plane of the bladder B along the longitudinal direction D2 of the subject S to generate ultrasound images of a plurality of frames. Each of the first contact position PP1 and the second contact position PP2 in Fig. 10 indicates that the ultrasound probe 2 is brought into contact with the body surface of the subject S such that the arrangement direction of the transducer array 2A of the ultrasound probe 2 extends along the lateral direction D1 and the longitudinal direction D2.

The maximum diameter measurement unit 13 measures the maximum diameter of a bladder region in a first group of ultrasound images of a plurality of frames corresponding to a plurality of tomographic planes along the lateral direction D1 of the subject S and the maximum diameter of a bladder region in a second group of ultrasound images of a plurality of frames corresponding to a plurality of tomographic planes along the longitudinal direction D2 of the subject S. Here, for example, the maximum diameter measurement unit 13 selects a representative frame where the area of the bladder region is maximized, among the first group of the ultrasound images of the plurality of frames corresponding to the plurality of tomographic plane along the lateral direction D1 of the subject S, and as shown in Fig. 11, measures the maximum diameter of a bladder region BR1 in an ultrasound image U1 of the selected representative frame, that is, the maximum diameter of the bladder B in the lateral direction D1 of the subject S. In this case, for example, the maximum diameter measurement unit 13 can measure the maximum diameter of the bladder region in the ultrasound image U1 by disposing a measurement line ML1 having the maximum length with two points on the contour of the bladder region BR1 as endpoints on the ultrasound image U1 and measuring the length of the disposed measurement line ML1. In an example shown in Fig. 11, the measurement line ML1 corresponding to the maximum diameter of the bladder region BR1 in a horizontal direction of the ultrasound image U1 is disposed on the ultrasound image U1.

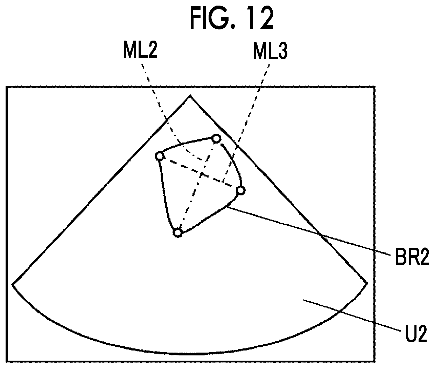

For example, the maximum diameter measurement unit 13 selects a representative frame where the area of the bladder region is maximized, among the second group of the ultrasound images of the plurality of frames corresponding to the plurality of tomographic planes along the longitudinal direction D2 of the subject S, and as shown in Fig. 12, measures the maximum diameter of a bladder region BR2 in the longitudinal direction of the subject S and the maximum diameter of the bladder region BR2 in the depth direction of the subject S from an ultrasound image U2 of the selected representative frame. In this case, for example, the maximum diameter measurement unit 13 can dispose a measurement line ML2 having the maximum length with two points on the contour of the bladder region BR1 as endpoints and a measurement line ML3 having the maximum length in a direction perpendicular to the measurement line ML2 with two points on the contour of the bladder region BR1 as endpoints on the ultrasound image U2, can measure the length of the disposed measurement line ML2 as the maximum diameter of the subject S in the longitudinal direction D2, and can measure the length of the measurement line ML3 as the maximum diameter of the subject S in the depth direction.

Here, as shown in Fig. 12, in the ultrasound image U2 representing the tomographic plane along the longitudinal direction D2 of the subject S, the bladder region BR2 is visualized in a tilted state due to a posture of the subject S and the tilt angle W of the ultrasound probe 2; however, the maximum diameter measurement unit 13 can obtain the maximum diameters of the bladder B in the depth direction and the longitudinal direction D2 of the subject S with excellent accuracy by setting the two measurement lines ML2 and ML3 perpendicular to each other for the bladder region BR2 and measuring the lengths of the set measurement lines ML2 and ML3.

The bladder volume calculation unit 15 calculates the volume of the bladder B of the subject S by applying each of the maximum diameters in the three directions of the bladder region BR1 or BR2 measured by the maximum diameter measurement unit 13, that is, the maximum diameter of the bladder B in the lateral direction D1 of the subject S, the maximum diameter of the bladder B in the longitudinal direction D2 of the subject S, and the maximum diameter of the bladder B in the depth direction of the subject S to any of the maximum diameter LX in the X direction, the maximum diameter LY in the Y direction, or the maximum diameter LZ in the Z direction of the ellipsoid E shown in Fig. 9 and calculating (LX × LY × LZ)/(6π). The bladder volume calculation unit 15 displays the calculated volume of the bladder B of the subject S as a urine volume in the bladder B on the display unit 7.

The notification unit 14 notifies a user of a determination result of the scanning success/failure determination unit 12, and the like. For example, in a case where determination is made that the scanning of the bladder B of the subject S with the ultrasound beam fails, the notification unit 14 notifies the user that the scanning with the ultrasound beam fails. In this case, the notification unit 14 may notify the user that the bladder B of the subject S is imaged again, along with the effect that the scanning of the bladder B of the subject S with the ultrasound beam fails. For example, in a case where the scanning of the bladder B of the subject S with the ultrasound beam is successful, the notification unit 14 may notify the user that the scanning with the ultrasound beam is successful. In a case where only one of the tomographic plane along the lateral direction or the tomographic plane along the longitudinal direction of the bladder B of the subject S is imaged, the notification unit 14 may notify the user that another tomographic plane of the bladder B is imaged.

The notification unit 14 can display information representing the notification to the user on the display unit 7 through the display controller 6. For example, in a case where the ultrasound diagnostic apparatus 1 comprises a speaker (not shown), the notification unit 14 can give notification to the user by generating voice through a speaker.

The apparatus controller 16 controls each unit of the ultrasound diagnostic apparatus 1 based on a program stored in advance in the storage unit 18 or the like and an input operation of the user through the input device 17.

The display controller 6 displays the ultrasound image generated by the image generation unit 5 of the image acquisition unit 8, and the like on the display unit 7 under the control of the apparatus controller 16.

The display unit 7 displays the ultrasound image generated by the image acquisition unit 8, and the like, and includes, for example, a display device, such as a liquid crystal display (LCD) or an organic electroluminescence display (organic EL display).

The input device 17 is provided for the user to perform an input operation, and can comprise a keyboard, a mouse, a trackball, a touch pad, a touch panel, and the like.

The storage unit 18 stores an operation program and the like of the ultrasound diagnostic apparatus 1, and a recording medium, such as a flash memory, an HDD, an SSD, an FD, an MO disc, an MT, a RAM, a CD, a DVD, an SD card, or a USB memory, a server, or the like can be used.

The processor 19 having the display controller 6, the image acquisition unit 8, the bladder extraction unit 10, the feature value calculation unit 11, the scanning success/failure determination unit 12, the maximum diameter measurement unit 13, the notification unit 14, the bladder volume calculation unit 15, and the apparatus controller 16 is configured of a central processing unit (CPU) and a control program causing the CPU to execute various kinds of processing. However, the processor 19 may be configured using a field programmable gate array (FPGA), a digital signal processor (DSP), an application specific integrated circuit (ASIC), a graphics processing unit (GPU), or other integrated circuits (ICs) or may be configured by combining the ICs.

The display controller 6, the image acquisition unit 8, the bladder extraction unit 10, the feature value calculation unit 11, the scanning success/failure determination unit 12, the maximum diameter measurement unit 13, the notification unit 14, the bladder volume calculation unit 15, and the apparatus controller 16 of the processor 19 can be configured to be partially or wholly integrated into one CPU or the like.

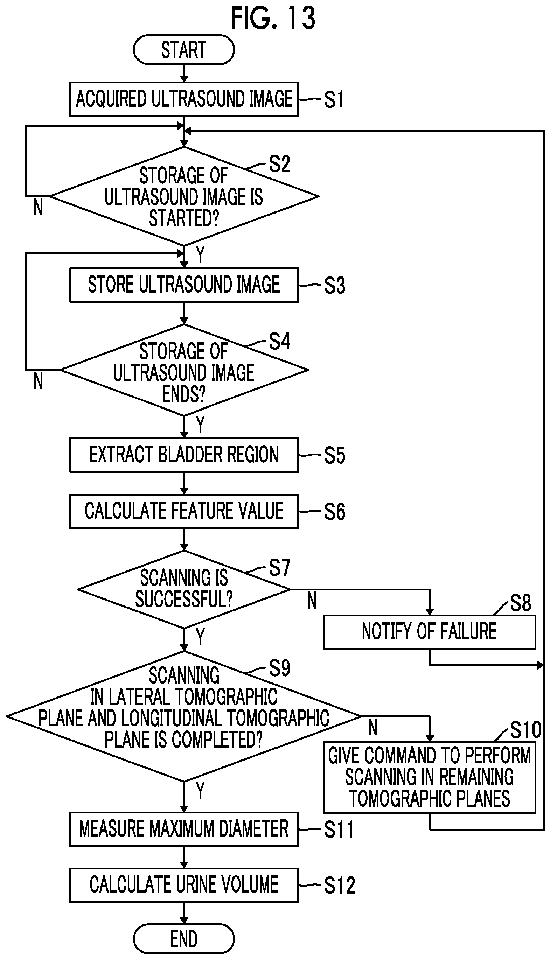

Next, the operation of the ultrasound diagnostic apparatus 1 in Embodiment 1 will be described in detail referring to a flowchart of Fig. 13.

First, in Step S1, as shown in Fig. 10, the ultrasound probe 2 is disposed at the first contact position PP1 of the subject S, and the ultrasound probe 2 is brought into contact with the body surface of the subject S by the user such that the ultrasound image U1 representing the tomographic plane of the bladder B along the lateral direction D1 of the subject S is acquired. The ultrasound beam is emitted from the ultrasound probe 2 into the subject S, and the first group of the ultrasound images U1 representing the tomographic plane of the bladder B along the lateral direction D1 of the subject S is acquired by the image acquisition unit 8. Subsequently, the first group of the ultrasound images U1 is continuously sequentially acquired by the image acquisition unit 8.

In Step S2, determination is made whether or not storage of the ultrasound image U1 acquired at present by the image acquisition unit 8 in the image memory 9 is started. For example, as a command to store the ultrasound image U1 acquired at present in the image memory 9 is issued by the user through the input device 17, the storage of the ultrasound image U1 is started. In a case where determination is made in Step S2 that the storage of the ultrasound image U1 is not started because the command to store the ultrasound image U1 is not issued by the user, or the like, the determination of Step S2 is performed again. In Step S2, in a case where determination is made that the storage of the ultrasound image U1 is started, the process progresses to Step S3.

In Step S3, the image generation unit 5 of the image acquisition unit 8 sends the first group of the generated ultrasound images U1 to the image memory 9 under the control of the apparatus controller 16. The image memory 9 stores the first group of the ultrasound images U1 sent from the image generation unit 5.

In subsequent Step S4, determination is made whether or not the storage of the ultrasound image U1 in the image memory 9 ends. For example, as a command to end the storage of the ultrasound image U1 at present is issued by the user through the input device 17, the storage of the ultrasound image U1 ends. In a case where determination is made in Step S4 that the storage of the ultrasound image U1 does not end and is continued because the command to end the storage of the ultrasound image U1 is not issued by the user, or the like, the process returns to Step S3, and the first group of the ultrasound image U1 newly acquired by the image acquisition unit 8 is stored in the image memory 9. In this manner, in Step S4, until determination is made that the storage of the ultrasound image U1 ends, the first group of the ultrasound image U1 is continuously stored in the image memory 9.

Here, while the first group of the ultrasound image U1 is continuously stored in the image memory 9, as shown in Fig. 4, the user changes the tilt angle W of the ultrasound probe 2 over the given angle range A with respect to the subject S while the contact position of the ultrasound probe 2 with the subject S is fixed at the first contact position PP1. With this, the first group of the ultrasound images U1 of the plurality of frames corresponding to the different tilt angles W of the ultrasound probe 2 is stored in the image memory 9.

In Step S4, in a case where determination is made that the storage of the ultrasound image U1 ends, the process progresses to Step S5.

In Step S5, the bladder extraction unit 10 extracts the bladder region BR1 from each of the first group of the ultrasound images U1 of the plurality of frames stored in the image memory 9 with the repetition of Steps S3 and S4. The bladder extraction unit 10 can extract bladder region BR1 in the ultrasound image U1 using, for example, a so-called pattern matching method.

In subsequent Step S6, the feature value calculation unit 11 calculates the feature value regarding the extracted bladder region BR1 on the first group of the ultrasound images U1 of the plurality of frames from which the bladder region BR1 is extracted in Step S5. For example, the feature value calculation unit 11 can calculate the area of the extracted bladder region BR1 as the feature value.

In Step S7, the scanning success/failure determination unit 12 analyzes change in feature value calculated in Step S6 between frames continuous in time series and determines whether or not the scanning of the bladder of the subject with the ultrasound beam is successful based on an analysis result. For example, in a case where the area of the bladder region BR1 is calculated as the feature value in Step S6, the scanning success/failure determination unit 12 determines that the scanning of the bladder B of the subject S with the ultrasound beam is successful in a case where a difference value of the areas of the bladder regions BR1 between any frames continuous in time series is smaller than a given threshold value, and determines that the scanning with the ultrasound beam fails in a case where a difference value of the feature values in at least one frame continuous in time series is equal to or greater than the threshold value.

That is, as shown in Fig. 6, in a case where the area of the bladder region BR1 calculated in Step S6 has continuity with respect to the time axis, the scanning success/failure determination unit 12 determines that the scanning of the bladder B of the subject S with the ultrasound beam is successful, and as shown in Figs. 7 and 8, in a case where there are the change point CP1, CP2, or CP3 where the area of the bladder region BR1 calculated in Step S6 changes extremely between frames continuous in time series, the scanning success/failure determination unit 12 determines that the scanning with the ultrasound beam fails.

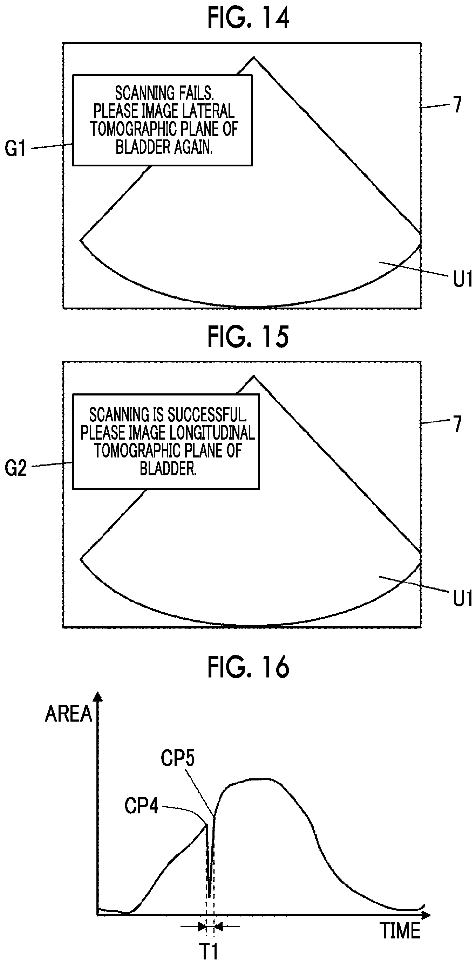

In Step S7, in a case where determination is made that the scanning of the bladder B of the subject S with the ultrasound beam fails, the process progresses to Step S8. In Step S8, the notification unit 14 notifies the user that the scanning of the bladder B of the subject S with the ultrasound beam fails. For example, as shown in Fig. 14, the notification unit 14 can display a guide panel G1 including information representing the effect that scanning fails and the effect that the tomographic plane of the bladder B of the subject S is imaged again, on the display unit 7. In an example shown in Fig. 14, the guide panel G1 includes text data "Scanning fails. Please image lateral tomographic plane of bladder again." and is displayed on the display unit 7 to be superimposed on the ultrasound image U1.

In this manner, in a case where the processing of Step S8 is completed, the process returns to Step S2, and determination is made whether or not the storage of the ultrasound image U1 is newly started. In a case where determination is made in Step S2 that the storage of the ultrasound image U1 is started, the process progresses to Step S3, the first group of ultrasound images U1 is newly stored in the image memory 9. In subsequent Step S4, Steps S3 and S4 are repeated until determination is made that the storage of the ultrasound image U1 ends, and the first group of the ultrasound images U1 of the plurality of frames is newly stored in the image memory 9.

In a case where determination is made in Step S4 that the storage of the ultrasound image U1 ends, the process progresses to Step S5, and the bladder region BR1 is extracted in the first group of the ultrasound images U1 of the plurality of frames newly stored in the image memory 9 by the repetition of Steps S3 and S4. In subsequent Step S6, the feature value regarding the bladder region BR1 extracted in the first group of the ultrasound images U1 of the plurality of frames is calculated, in Step S7, change in feature value between continuous frames of the first group of the ultrasound images U1 of the plurality of frames is analyzed, and determination is made whether or not the scanning of the bladder B of the subject S with the ultrasound beam is successful based on an analysis result. In Step S7, in a case where determination is made that the scanning of the bladder B of the subject S with the ultrasound beam fails, the process progresses to Step S8, the user is notified that the scanning with the ultrasound beam fails, and the process returns to Step S2.

In this way, in Step S7, until determination is made that the scanning of the bladder B of the subject S with the ultrasound beam is successful, the processing of Steps S2 to S8 is repeated, and determination is made whether or not new scanning of the bladder B of the subject S with the ultrasound beam is successful. With this, the first group of the ultrasound images U1 of the plurality of frames with the bladder region BR1 clearly shown in all the whole ultrasound images U1 is obtained.

In Step S7, in a case where determination is made that the scanning of the bladder B of the subject S with the ultrasound beam is successful, the process progresses to Step S9. In Step S9, determination is made whether or not both the scanning with the ultrasound beam in the tomographic plane along the lateral direction of bladder B of the subject S and the scanning with the ultrasound beam in the tomographic plane along the longitudinal direction of the bladder B are completed. At the moment, only the scanning with the ultrasound beam in the tomographic plane along the lateral direction of the bladder B of the subject S is completed, the scanning with the ultrasound beam in the tomographic plane along the longitudinal direction of the bladder B of the subject S is not completed. Thus, in Step S9, determination is made that the scanning of the ultrasound beam in two tomographic planes of the tomographic plane along the lateral direction and the tomographic plane along the longitudinal direction of the bladder B of the subject S is not completed, and the process progresses to Step S10.

In Step S10, the notification unit 14 gives a command to the user to the effect that the scanning of the ultrasound beam is not completed and the scanning with the ultrasound beam in the remaining tomographic plane is performed. For example, as shown in Fig. 15, the notification unit 14 can display a guide panel G2 including information representing the effect that scanning is successful and the effect that the remaining tomographic plane is imaged, on the display unit 7. In an example shown in Fig. 15, the guide panel G2 includes text data "Scanning is successful. Please image longitudinal tomographic plane of bladder." and is displayed on the display unit 7 to be superimposed on the ultrasound image U1.

In compliance with the command by the notification unit 14 in Step S10, as shown in Fig. 10, the user disposes the ultrasound probe 2 at the second contact position PP2 of the subject S and brings the ultrasound probe 2 into contact with the body surface of the subject S such that a second group of an ultrasound image U2 representing a tomographic plane along the longitudinal direction of the bladder B of the subject, that is, a tomographic plane of the bladder B along the longitudinal direction D2 of the subject S is acquired. In this state, the ultrasound beam is emitted from the ultrasound probe 2 into the subject S, and the second group of the ultrasound image U2 representing the tomographic plane of the bladder B along the longitudinal direction D2 of the subject S is acquired by the image acquisition unit 8.

In this manner, the process returns from Step S10 to Step S2, and the processing of Steps S2 to S8 is executed using the ultrasound probe 2 disposed at the second contact position PP2 of the subject S. In regards to Steps S2 to S8, similarly to a case where the scanning with the ultrasound beam is performed in the tomographic plane of the bladder B along the lateral direction D1 of the subject S, the second group of the ultrasound images U2 of the plurality of frames is newly stored in the image memory 9, and the bladder region BR2 is extracted in the second group of the ultrasound images U2 of the plurality of frames newly stored in the image memory 9.

The feature value regarding the extracted bladder region BR2 is calculated, determination is made whether or not the scanning of the bladder B of the subject S with the ultrasound beam is successful based on change in calculated feature value between continuous frames, and the processing of Steps S2 to S8 is repeated until determination is made that the scanning with the ultrasound beam is successful. With this, the second group of the ultrasound images U2 of the plurality of frames with the bladder region BR2 clearly shown in all the ultrasound images U2 is obtained.

In Step S7, in a case where determination is made that the scanning with the ultrasound beam in the tomographic plane of the bladder B along the longitudinal direction D2 of the subject S is successful, the process progresses to Step S9.

In Step S9, determination is made whether or not both the scanning with the ultrasound beam in the tomographic plane of the bladder B along the lateral direction D1 of the subject S and the scanning with the ultrasound beam in the tomographic plane of the bladder B along the longitudinal direction of the subject S are completed. At the moment, both the scanning with the ultrasound beam in the tomographic plane of the bladder B along the lateral direction D1 of the subject S and the scanning with the ultrasound beam in the tomographic plane of the bladder B along the longitudinal direction D2 of the subject S are completed, and thus, the process progresses to Step S11.

In Step S11, the maximum diameter measurement unit 13 measures the maximum diameter of the bladder region BR1 in the first group of the ultrasound images U1 of the plurality of frames corresponding to the plurality of tomographic planes of the bladder B along the lateral direction D1 of the subject S and the maximum diameter of the bladder region BR2 in the second group of the ultrasound images U2 of the plurality of frames corresponding to the plurality of tomographic planes of the bladder B along the longitudinal direction D2 of the subject S.

For example, in a case where the area of the bladder region BR1 is calculated as the feature value in Step S6, the maximum diameter measurement unit 13 selects a representative frame where the area of the bladder region BR1 is maximized, among the first group of the ultrasound images U1 of the plurality of frames corresponding to the plurality of tomographic planes along the lateral direction of the subject S, for example, with reference to the area of the bladder region BR1 calculated in Step S6, and as shown in Fig. 11, measures the maximum diameter of the bladder region BR1 in the lateral direction of the ultrasound image U1 of the selected representative frame as the maximum diameter of the bladder B of the subject S in the lateral direction D1 of the subject S. In this case, the maximum diameter measurement unit 13 can measure the maximum diameter of the bladder region BR1 in the ultrasound image U1, for example, by disposing the measurement line ML1 having the maximum length in the lateral direction D1 with the two points on the contour of the bladder region BR1 as endpoints on the ultrasound image U1 and measuring the length of the disposed measurement line ML1.

The maximum diameter measurement unit 13 selects a representative frame where the area of the bladder region BR2 is maximized, among the second group of the ultrasound images U2 of the plurality of frames corresponding to the plurality of tomographic planes along the longitudinal direction of the subject S, for example, with reference to the area of the bladder region BR2 calculated in Step S6, and as shown in Fig. 12, measures the maximum diameter of the bladder B in the longitudinal direction D2 of the subject S and the maximum diameter of the bladder B in the depth direction of the subject S from the bladder region BR2 in the ultrasound image U2 of the selected representative frame. In this case, for example, the maximum diameter measurement unit 13 can dispose the measurement line ML2 having the maximum length with the two points on the contour of the bladder region BR2 as endpoints and the measurement line ML3 having the maximum length in the direction perpendicular to the measurement line ML2 with the two points on the contour of the bladder region BR2 as endpoints on the ultrasound image U2, can measure the length of the disposed measurement line ML2 as the maximum diameter of the bladder B in the longitudinal direction D2 of the subject S, and can measure the length of the measurement line ML3 as the maximum diameter of the bladder B in the depth direction of the subject S.

In subsequent Step S12, the bladder volume calculation unit 15 calculates the volume of the bladder B of the subject S by applying the maximum diameters of the bladder B in the lateral direction D1, the longitudinal direction D2, and the depth direction of the subject S measured in Step S11 to the maximum diameter LX in the X direction, the maximum diameter LY in the Y direction, and the maximum diameter LZ in the Z direction of the ellipsoid E shown in Fig. 9, denoting the circular constant as π, and calculating (LX × LY × LZ)/(6π). Though not shown, the bladder volume calculation unit 15 displays the calculated volume of the bladder B of the subject S as the urine volume of the bladder B of the subject S on the display unit 7.

In a case where the processing of Step S12 is completed, the operation of the ultrasound diagnostic apparatus 1 ends.

From the above description, since the scanning success/failure determination unit 12 determines whether or not the scanning of the bladder B of the subject S with the ultrasound beam is successful, the maximum diameter measurement unit 13 measures the maximum diameters in the lateral direction D1, the longitudinal direction D2, and the depth direction of the subject S from the first group of the ultrasound images U1 of the plurality of frames with the bladder region BR1 clearly visualized in all the ultrasound images U1 and the second group of the ultrasound images U2 of the plurality of frames with the bladder region BR2 clearly visualized in all the ultrasound images U2 with excellent accuracy, and the bladder volume calculation unit 15 calculates the volume of the bladder B of the subject S as the urine volume in the bladder B based on the measured maximum diameters in the lateral direction, the longitudinal direction, and in the depth direction of the bladder B of the subject S. For this reason, with the ultrasound diagnostic apparatus 1 according to Embodiment 1 of the invention, it is possible to measure the urine volume in the bladder B of the subject S with excellent accuracy.

In Step S2, although an example where the storage of the ultrasound image U1 or U2 in the image memory 9 is started with the command to store the ultrasound image U1 or U2 issued by the user through the input device 17 as a trigger, and in Step S4, the storage of the ultrasound image U1 or U2 ends with the command to end the storage of the ultrasound image U1 or U2 issued by the user through the input device 17 as a trigger has been exemplified, the trigger for starting the storage of the ultrasound image U1 or U2 and the trigger for ending the storage of the ultrasound image U1 or U2 are not limited thereto.

For example, in Step S2, the storage of the ultrasound image U1 or U2 can be automatically started instead of performing determination about whether or not the storage of the ultrasound image U1 or U2 is started.

The storage of the ultrasound image U1 or U2 may end in a case where a given time, for example, 15 seconds elapse after the storage of the ultrasound image U1 or U2 is started in Step S2.

Alternatively, for example, a probe contact determination unit that analyzes the acquired ultrasound image U1 or U2 to determine whether or not the ultrasound probe 2 is in contact with the subject S or is apart from the subject S may be provided in the ultrasound diagnostic apparatus 1, the storage of the ultrasound image U1 or U2 may start with determination by the probe contact determination unit that the ultrasound probe 2 is in contact with the subject S, as a trigger, and the storage of the ultrasound image U1 or U2 may end with determination by the probe contact determination unit that the ultrasound probe 2 is apart from the subject S, as a trigger.

In a case where determination is made in Step S7 that the scanning of the bladder B of the subject S with the ultrasound beam fails, the effect that the scanning with the ultrasound beam fails is notified in Step S8, the process returns to Step S2, and determination is made whether or not to newly store the ultrasound image U1 or U2; however, even in a case where determination is made that the scanning with the ultrasound beam fails, the urine volume in the bladder B of the subject S may be calculated using the ultrasound image U1 or U2 of the plurality of frames already stored in the image memory 9.

For example, in a case where the area of the bladder region BR1 or BR2 is calculated as the feature value by the feature value calculation unit 11, as shown in Fig. 16, the maximum diameter measurement unit 13 can select a frame where the area is maximized, as a representative frame from the ultrasound images U1 or U2 of a plurality of frames excluding ultrasound images of frames corresponding to a section T1 between a change point CP4 and a change point CP5 where the calculated area changes extremely with respect to the time axis, to measure the maximum diameter of the bladder region BR1 or BR2. The bladder volume calculation unit 15 calculates the volume of the bladder B of the subject S as the urine volume in the bladder B of the subject S based on the maximum diameters of the bladder region BR1 or BR2 calculated by the maximum diameter measurement unit 13 in this manner.

With this, it is possible to calculate the urine volume in the bladder B of the subject S using only the ultrasound images U1 or U2 of the plurality of frames where the feature value changes continuously, and thus, it is possible to improve the measurement accuracy of the urine volume.

Although an example where the feature value calculation unit 11 calculates the area of the bladder region BR1 or BR2 in the ultrasound image U1 or U2 as the feature value has been exemplified, the feature value calculation unit 11 may calculate the diameter of the bladder region BR1 or BR2 as the feature value. For example, in a case where the ultrasound probe 2 is disposed at the first contact position PP1 shown in Fig. 10 and the tomographic plane of the bladder B along the lateral direction D1 of the subject S is visualized in the ultrasound image U1, as shown in Fig. 11, the feature value calculation unit 11 can calculate a diameter of the bladder region BR1 represented by the measurement line ML1 as the diameter of the bladder region BR1. Here, the diameter of the bladder region BR1 represented by the measurement line ML1 is the maximum diameter of the bladder region BR1 in the lateral direction of the ultrasound image U1 and corresponds to the maximum diameter of the bladder B of the subject S in the lateral direction D1 of the subject S.

For example, in a case where the ultrasound probe 2 is disposed at the second contact position PP2 shown in Fig. 10, and the tomographic plane of the bladder B along the longitudinal direction D2 of the subject S is visualized in the ultrasound image U2, as shown in Fig. 12, the feature value calculation unit 11 can calculate a diameter of the bladder region BR2 represented by the measurement line ML2 or a diameter of the bladder region BR2 represented by the measurement line ML3 as the diameter of the bladder region BR2. Here, the diameter of the bladder region BR2 represented by the measurement line ML2 is the maximum diameter of the bladder region BR2, and corresponds to the maximum diameter of the bladder B of the subject S in the depth direction of the subject S. The diameter of the bladder region BR2 represented by the measurement line ML3 is the maximum diameter of the bladder region BR2 in the direction perpendicular to the measurement line ML2, and corresponds to the maximum diameter of the bladder B of the subject S in the longitudinal direction D2 of the subject S.

For example, in a case where the feature value calculation unit 11 calculates the diameter of the bladder region BR1 or BR2 in the ultrasound image U1 or U2 as the feature value, the scanning success/failure determination unit 12 can determine that the scanning of the bladder B of the subject S with the ultrasound beam is successful in a case where a difference of the diameters of the bladder region BR1 or BR2 between frames continuous in time series is smaller than a given threshold value, and can determine that the scanning with the ultrasound beam fails in a case where the difference of the diameters of the bladder region BR1 or BR2 in at least one frame continuous in time series is equal to or greater than the threshold value. The maximum diameter measurement unit 13 can select a frame where the diameter of the bladder region BR1 or BR2 is maximized, as the representative frame among the ultrasound images of the plurality of frames, for example, with reference to the diameter of the bladder region BR1 or BR2 calculated by the feature value calculation unit 11, and can set the diameters measured from the bladder region BR1 or BR2 in the ultrasound image of the selected frame as the maximum diameters of the bladder B of the subject S in the lateral direction D1, the longitudinal direction D2, and the depth direction of the subject S. The bladder volume calculation unit 15 calculates the volume of the bladder B as the urine volume in the bladder B based on the maximum diameters of the bladder B in the lateral direction D1, the longitudinal direction D2, and the depth direction of the subject S measured by the maximum diameter measurement unit 13.

In this way, even in a case where the feature value calculation unit 11 calculates the diameter of the bladder region BR1 or BR2 in the ultrasound image U1 or U2 as the feature value, similarly to the case where the area of the bladder region BR1 or BR2 is calculated as the feature value, the scanning success/failure determination unit 12 determines whether or not the scanning of the bladder B of the subject S with the ultrasound beam is successful, and the urine volume in the bladder B of the subject S is calculated from the ultrasound images U1 or U2 of the plurality of frames where the scanning with the ultrasound beam is successful. Thus, it is possible to measure the urine volume in the bladder B of the subject S with excellent accuracy.

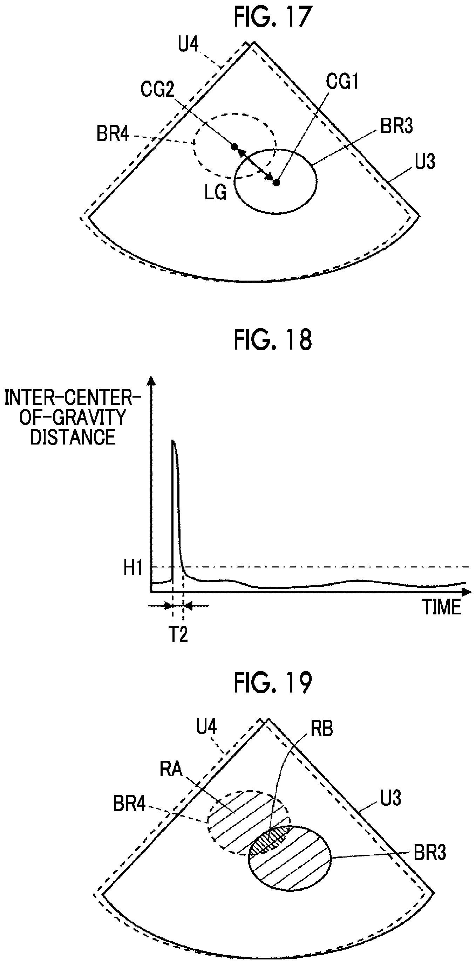

For example, the feature value calculation unit 11 may calculate a position of the bladder region BR1 or BR2 in the ultrasound image U1 or U2 as a feature value. For example, as shown in Fig. 17, the feature value calculation unit 11 can calculate a position of a center of gravity CG1 of a bladder region BR3 in an ultrasound image U3 as a feature value. In this case, from an ultrasound image U3 and an ultrasound image U4 continuous in time series, the scanning success/failure determination unit 12 can calculate an inter-center-of-gravity distance LG between a center of gravity CG1 of a bladder region BR3 of the ultrasound image U3 and a center of gravity CG2 of a bladder region BR4 of the ultrasound image U4. Here, in Fig. 17, for description, although the ultrasound image U3 and the ultrasound image U4 are disposed to slightly deviate from each other, actually, the inter-center-of-gravity distance LG between the center of gravity CG1 of the bladder region BR3 and the center of gravity CG2 of the bladder region BR4 is calculated in a state in which the ultrasound image U3 and the ultrasound image U4 are disposed to overlap each other without deviating from each other.

The scanning success/failure determination unit 12 can determine that the scanning of the bladder B of the subject S with the ultrasound beam is successful in a case where the inter-center-of-gravity distance LG between any frames continuous in time series is smaller than a given threshold value, and can determine that the scanning with the ultrasound beam fails in a case where the inter-center-of-gravity distance LG in at least one frame continuous in time series is equal to or greater than the given threshold value. For example, as shown in Fig. 18, in a case where there is the inter-center-of-gravity distance LG equal to or greater than a given threshold value H1 among a plurality of inter-center-of-gravity distances LG calculated from the ultrasound images of the plurality of frames, the scanning success/failure determination unit 12 determines that the scanning of the bladder B of the subject S with the ultrasound beam fails.

The scanning success/failure determination unit 12 may perform both of a success/failure determination regarding the scanning with the ultrasound beam using the inter-center-of-gravity distance LG between the frames continuous in time series and a success/failure determination regarding the scanning with the ultrasound beam using the difference of the areas of the bladder region BR3 or the difference of the diameters of the bladder region BR3 between the frames continuous in time series, may make final determination that the scanning with the ultrasound beam is successful in a case where determination is made that the scanning with the ultrasound beam is successful through both the success/failure determinations, and may make final determination that the scanning with the ultrasound beam fails in a case where determination is made that the scanning with the ultrasound beam fails through at least one success/failure determination. With this, it is possible to acquire ultrasound images U3 and U4 of a plurality of frames where the scanning of the bladder B of the subject S with the ultrasound beam is successful, with excellent accuracy, and thus, it is possible to improve the measurement accuracy of the urine volume in the bladder B of the subject S.

Even in a case where the scanning success/failure determination unit 12 determines that the scanning of the bladder B of the subject S with the ultrasound beam fails, for example, as shown in Fig. 18, the maximum diameter measurement unit 13 may measure the maximum diameter of the bladder B in the lateral direction D1, the longitudinal direction D2, and the depth direction of the subject S from the ultrasound images of the plurality of frames other than frames in a section T2 where the inter-center-of-gravity distance LG is greater than the given threshold value H1. In this case, the bladder volume calculation unit 15 calculates the volume of the bladder B of the subject S using only the ultrasound images of the frames where the scanning with the ultrasound beam is successful, and thus, it is possible to improve the measurement accuracy of the urine volume in the bladder B.

For example, as shown in Fig. 19, the feature value calculation unit 11 may calculate the areas and the positions of the bladder regions BR3 and BR4 in the ultrasound images U3 and U4 as the feature value. In this case, the scanning success/failure determination unit 12 can calculate a ratio of an area of a region RB where the bladder region BR3 and the bladder region BR4 overlap each other to an area of a region RA occupied by at least one of the bladder region BR3 in the ultrasound image U3 or the bladder region BR4 in the ultrasound image U4 from the ultrasound image U3 and the ultrasound image U4 continuous in time series. Here, in Fig. 19, for description, although the ultrasound image U3 and the ultrasound image U4 are disposed to slightly deviate from each other, actually, the ratio of the area of the region RB to the area of the region RA is calculated in a state in which the ultrasound image U3 and the ultrasound image U4 overlap each other without deviating from each other.

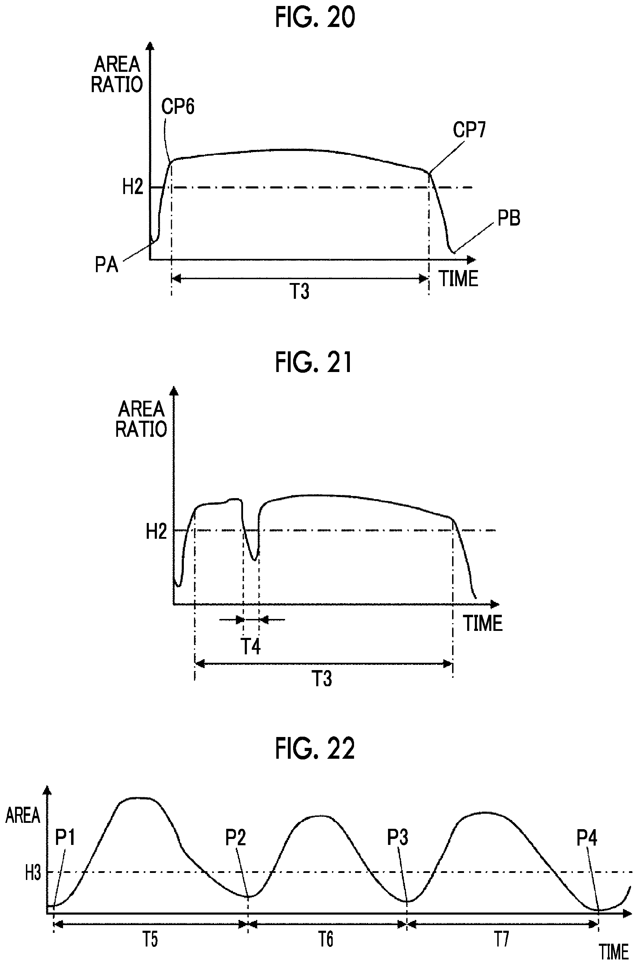

Here, as shown in Fig. 4, while the ultrasound probe 2 is tilted in the given angle range A, in a case where the ultrasound probe 2 is tilted from a state in which the scanning plane PS1 passes through the center C of the bladder B of the subject S, the scanning plane PS1 is gradually apart from the center C of the bladder B. In this case, the areas of the bladder regions BR3 and BR4 in the ultrasound images U3 and U4 gradually decrease and approach 0. For this reason, in a case where the ultrasound probe 2 is tilted such that the tilt angle W of the ultrasound probe 2 changes from a lower limit value to an upper limit value of the angle range A, for example, as shown in Fig. 20, the ratio of the area of the region RB to the area of the region RA has minimum values PA and PB and change points CP6 and CP7 in both end portions in time series. For this reason, the scanning success/failure determination unit 12 determines that the scanning of the bladder B of the subject S with the ultrasound beam is successful, for example, in a case where the ratio of the area of the region RB to the area of the region RA is equal to or greater than a given threshold value H2 in a section T3 between the change point CP6 and the change point CP7 of both end portions in time series as shown in Fig. 20, and can determine that the scanning with the ultrasound beam fails in a case where the ratio of the area of the region RB to the area of the region RA is smaller than the given threshold value H2 as shown in Fig. 21.

The scanning success/failure determination unit 12 can perform both of a success/failure determination regarding the scanning with the ultrasound beam using the ratio of the area of the region RB to the area of the region RA between the frames continuous in time series and a success/failure determination regarding the scanning with the ultrasound beam using the difference of the areas of the bladder region BR3 or the difference of the diameters of the bladder region BR3 between the frames continuous in time series, can make final determination that the scanning with the ultrasound beam is successful in a case where determination is made that the scanning with the ultrasound beam is successful through both the success/failure determinations, and can make final determination that the scanning with the ultrasound beam fails in a case where determination is made that the scanning with the ultrasound beam fails. With this, it is possible to acquire ultrasound images U3 and U4 of a plurality of frames where the scanning of the bladder B of the subject S with the ultrasound beam is successful, with excellent accuracy, and thus, it is possible to improve the measurement accuracy of the urine volume in the bladder B of the subject S.

Even in a case where the scanning success/failure determination unit 12 determines that the scanning of the bladder B of the subject S with the ultrasound beam fails, for example, as shown in Fig. 21, the maximum diameter measurement unit 13 may measure the maximum diameters of the bladder B in the lateral direction D1, the longitudinal direction D2, and the depth direction of the subject S from the ultrasound images of the plurality of frames other than frames in a section T4 where the ratio of the area of the region RB to the area of the region RA is smaller than the given threshold value. In this case, the bladder volume calculation unit 15 calculates the volume of the bladder B of the subject S using only the ultrasound images of the frames where the scanning with the ultrasound beam is successful, and thus, it is possible to improve the measurement accuracy of the urine volume in the bladder B.

In Embodiment 1, although, first, to obtain the first group of the ultrasound images U1 for visualizing the tomographic plane of the bladder B along the lateral direction D1 of the subject S, as shown in Fig. 10, the ultrasound probe 2 is disposed at the first contact position PP1, and next, to obtain the second group of the ultrasound images U2 for visualizing the tomographic plane of the bladder B along the longitudinal direction D2 of the subject S, the ultrasound probe 2 is disposed at the second contact position PP2, first, the ultrasound probe 2 may be disposed at the second contact position PP2 to acquire the second group of the ultrasound images U2, and next, the ultrasound probe 2 may be disposed at the first contact position PP1 to acquire the first group of the ultrasound images U1.

For example, in starting the operation of the ultrasound diagnostic apparatus 1, the notification unit 14 may notify that the tomographic plane of the bladder B in the lateral direction D1 of the subject S is imaged or that the tomographic plane of the bladder B in the longitudinal direction D2 of the subject S is imaged. Though not shown, for example, the notification unit 14 can display information representing that the tomographic plane of the bladder B in the lateral direction D1 of the subject S is imaged or information representing that the tomographic plane of the bladder B in the longitudinal direction D2 of the subject S is imaged, on the display unit 7.

### Embodiment 2

In Embodiment 1, as shown in Fig. 4, although the single scanning with the ultrasound beam is performed in which the tilt angle W of the ultrasound probe 2 is changed from the lower limit value to the upper limit value or from the upper limit value to the lower limit value of the given angle range A by rotating the ultrasound probe 2 around the rotation axis R while the contact position of the ultrasound probe 2 with the subject S is fixed, a plurality of times of scanning with the ultrasound beam may be continuously performed.

Even in this case, similarly to a case where only the single scanning with the ultrasound beam is performed, the scanning success/failure determination unit 12 determines that a plurality of times of the scanning of the bladder B of the subject S with the ultrasound beam are successful in a case where change in feature value calculated by the feature value calculation unit 11 in the ultrasound images U1 or U2 of the plurality of frames is continuous in time series, and determines that the plurality of times of the scanning with the ultrasound beam fail in a case where the change in feature value calculated in the ultrasound images U1 or U2 of the plurality of frames is discontinuous in time series.

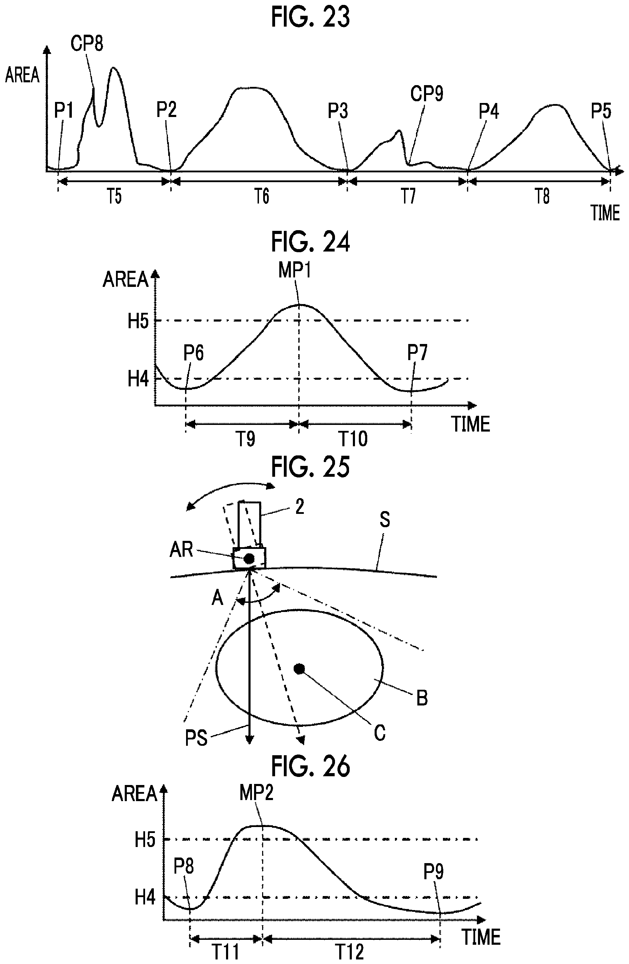

For example, in a case where the feature value calculation unit 11 calculates the area of the bladder region BR1 or BR2 in the ultrasound images U1 or U2 of the plurality of frames, the scanning success/failure determination unit 12 can determine that the plurality of times of the scanning of the bladder B of the subject S with the ultrasound beam are successful in a case where the value of the area is continuous with respect to the time axis and the difference value of the areas of the bladder region BR1 or BR2 between any frames continuous in time series is smaller than the given threshold value, as shown in Fig. 22. The scanning success/failure determination unit 12 can determine that the plurality of times of the scanning of the bladder B of the subject S with the ultrasound beam fail in a case where change in area of the bladder region BR1 or BR2 in time series is discontinuous and the difference value of the areas of the bladder region BR1 or BR2 in at least one frame continuous in time series is equal to or greater than the threshold value, as shown in Fig. 23. In an example shown in Fig. 23, the area of the bladder region BR1 or BR2 of the ultrasound images U1 or U2 of the plurality of frames has change points CP8 and CP9 where the difference of the areas of the bladder region BR1 or BR2 between preceding and following frames is equal to or greater than a given threshold value.

In this manner, even in a case where the plurality of times of the scanning with the ultrasound beam are performed, similarly to a case where only the single scanning with the ultrasound beam is performed, the scanning success/failure determination unit 12 determines whether or not the scanning of the bladder B of the subject S with the ultrasound beam is successful. Therefore, the urine volume in the bladder B of the subject S is measured using only the ultrasound images U1 or U2 of the frames where the scanning with the ultrasound beam is successful, and thus, it is possible to improve the measurement accuracy of the urine volume in the bladder B.

Although the scanning success/failure determination unit 12 determines whether or not the plurality of times of the scanning with the ultrasound beam are successful depending on whether or not the ultrasound images U1 or U2 of the plurality of frames acquired by the image acquisition unit 8 while the plurality of times of the scanning of the bladder B of the subject S with the ultrasound beam are performed are continuous as a whole, the scanning success/failure determination unit 12 may perform determination about whether or not the scanning with the ultrasound beam is successful for each scanning with the ultrasound beam among the plurality of times of the scanning with the ultrasound beam.

In this case, for example, in a case where the feature value calculation unit 11 calculates the area of the bladder region BR1 or BR2 in the ultrasound images U1 or U2 of the plurality of frames, as shown in Fig. 22, the scanning success/failure determination unit 12 detects minimum values P1, P2, P3, and P4 the area of the bladder region BR1 or BR2 smaller than a given threshold value H3, and estimates each of a section T5 between a position on the time axis indicating the minimum value P1 and a position on the time axis indicating the minimum value P2, a section T6 between a position on the time axis indicating the minimum value P2 and a position on the time axis indicating the minimum value P3, and a section T7 between a position on the time axis indicating the minimum value P3 and a position on the time axis indicating the minimum value P4 as a section where the single scanning with the ultrasound beam is performed. The scanning success/failure determination unit 12 determines whether or not the scanning of the bladder B of the subject S with the ultrasound beam is successful on each of the sections T5, T6, and T7 estimated in this manner. In an example shown in Fig. 22, the area of the bladder region BR1 or BR2 is continuous with respect to the time axis in all the sections T5, T6, and T7, and thus, the scanning success/failure determination unit 12 determines that the scanning with the ultrasound beam is successful on each of the sections T5, T6, and T7.

In this case, the maximum diameter measurement unit 13 can select an ultrasound image of one representative frame where the area of the bladder region BR1 or BR2 is maximized, among the ultrasound images U1 or U2 of a plurality of frames corresponding to the sections T5, T6, and T7, for example, and measure the maximum diameter of the bladder region BR1 or BR2 from the selected ultrasound image of the representative frame. The maximum diameter measurement unit 13 can select an ultrasound image of a first representative frame where the area of the bladder region BR1 or BR2 is maximized, in the ultrasound images of a plurality of frames corresponding to the section T5, an ultrasound image of a second representative frame where the area of the bladder region BR1 or BR2 is maximized in the ultrasound images of a plurality of frames corresponding to the section T6, and an ultrasound image of a third representative frame where the area of the bladder region BR1 or BR2 is maximized, in the ultrasound images of a plurality of frames corresponding to the section T7, and can measure the maximum diameter of the bladder region BR1 or BR2 from each of the ultrasound images of the selected first, second, and third representative frames. The maximum diameter measurement unit 13 can measure the final maximum diameter of the bladder region BR1 or BR2 by averaging the maximum diameters of the bladder region BR1 or BR2 measured from the ultrasound images of the first, second, and third representative frames.

For example, as shown in Fig. 23, in a case where the scanning success/failure determination unit 12 estimates four sections T5, T6, T7, and T8, the area of the bladder region BR1 or BR2 is continuous with respect to the time axis in the sections T6 and T8, and the change of the area of the bladder region BR1 or BR2 in time series is discontinuous in the sections T5 and T7, the scanning success/failure determination unit 12 determines that the scanning with the ultrasound beam is successful on the sections T6 and T8, and determines that the scanning with the ultrasound beam fails on the sections T5 and T7.

In this case, the maximum diameter measurement unit 13 can measure the maximum diameter of the bladder region BR1 or BR2 using the ultrasound images U1 or U2 of the plurality of frames corresponding to the sections T6 and T8 where the scanning success/failure determination unit 12 determines that the scanning of the bladder B of the subject S with the ultrasound beam is successful, among the ultrasound images U1 or U2 of the plurality of frames corresponding to the sections T5, T6, T7, and T8. For example, the maximum diameter measurement unit 13 can select an ultrasound image of one representative frame where the area of the bladder region BR1 or BR2 is maximized, among the ultrasound images U1 or U2 of the plurality of frames corresponding to the sections T6 and T8, and can measure the maximum diameter of the bladder region BR1 or BR2 from the selected ultrasound image of the representative frame.

The maximum diameter measurement unit 13 can select an ultrasound image of a first representative frame where the area of the bladder region BR1 or BR2 is maximized, among the ultrasound images U1 or U2 of a plurality of frames corresponding to the section T6 and an ultrasound image of a second representative frame where the area of the bladder region BR1 or BR2 is maximized, among the ultrasound images U1 or U2 of a plurality of frames corresponding to the section T8, and can measure the maximum diameter of the bladder region BR1 or BR2 from each of the ultrasound images of the first and second representative frames. The maximum diameter measurement unit 13 can measure the final maximum diameter of the bladder region BR1 or BR2 by averaging the maximum diameters of the bladder region BR1 or BR2 measured from the ultrasound images of the first and second representative frames.

In this manner, the scanning success/failure determination unit 12 estimates the sections T5 to T8 on the time axis each corresponding to the single scanning with the ultrasound beam from the plurality of times of the scanning with the ultrasound beam, and determines whether or not the scanning of the bladder B of the subject S with the ultrasound beam is successful on each of the estimated sections T5 to T8. Accordingly, for example, even in a case where the change in feature value is discontinuous in the ultrasound images U1 or U2 of the plurality of frames acquired while the plurality of times of the scanning with the ultrasound beam are performed, it is possible to measure the urine volume in the bladder B of the subject S using only the ultrasound images U1 or U2 of a plurality of frames where the bladder region BR1 or BR2 is clearly visualized.

In Embodiment 2, although the scanning success/failure determination unit 12 determines whether or not the scanning of the bladder B of the subject S with the ultrasound beam is successful based on the change in area of the bladder region BR1 or BR2 in the ultrasound image U1 or U2 between the frames continuous in time series, the scanning success/failure determination unit 12 may determine whether or not the scanning with the ultrasound beam is successful based on the inter-center-of-gravity distance LG of the bladder region BR1 or BR2 between the frames continuous in time series. The scanning success/failure determination unit 12 may determine whether or not the scanning with the ultrasound beam is successful based on the ratio of the area of the region RB where the bladder regions BR1 or BR2 overlap each other between the frames continuous in time series to the area of the region RA occupied by at least one of the bladder regions BR1 or BR2. Even in such cases, it is possible to measure the urine volume in the bladder B of the subject S using only the ultrasound images U1 and U2 of the plurality of frames where the bladder region BR1 or BR2 is clearly visualized.

### Embodiment 3

As the bladder B is regarded as the ellipsoid E shown in Fig. 9, the volume of the bladder B of the subject S is calculated based on the maximum diameters of the bladder B in the lateral direction D1, the longitudinal direction D2, and the depth direction of the subject S corresponding to the maximum diameter LX in the X direction, the maximum diameter LY in the Y direction, and the maximum diameter LZ in the Z direction of the ellipsoid E. Thus, in a case where the scanning with the ultrasound beam is performed, to measure the maximum diameter of the bladder B in the lateral direction D1, the longitudinal direction D2, and the depth direction of the subject S with excellent accuracy and to calculate the volume of the bladder B with excellent accuracy, as shown in Fig. 4, it is desirable that the ultrasound probe 2 is positioned directly above the center C of the bladder B of the subject S.

In this way, in a case where the ultrasound probe 2 is positioned directly above the center of the bladder B of the subject S, and the ultrasound probe 2 is tilted at a constant speed over the given angle range A, for example, the area of the bladder region BR1 or BR2 calculated as the feature value by the feature value calculation unit 11 in the ultrasound images U1 or U2 of the plurality of frames has symmetry in a time axis direction as shown in Fig. 24. For this reason, in a case where a distribution of the feature values in the ultrasound images U1 or U2 of the plurality of frames with respect to the time axis has symmetry in the time axis direction, determination can be made that the ultrasound probe 2 is positioned directly above the center C of the bladder B of the subject S.

In Embodiments 1 and 2, although the scanning success/failure determination unit 12 determines whether or not the scanning of the bladder B of the subject S with the ultrasound beam is successful depending on whether or not the feature value, such as the area of the bladder region BR1 or BR2, calculated by the feature value calculation unit 11 in the ultrasound images U1 or U2 of the plurality of frames is continuous in time series, the scanning success/failure determination unit 12 can further determine whether or not the distribution of the feature values in the ultrasound images U1 and U2 of the plurality of frames with respect to the time axis has symmetry with respect to the time axis direction, and can determine whether or not the scanning with the ultrasound beam is successful in consideration of the symmetry of the feature values in the ultrasound images U1 or U2 of the plurality of frames with respect to the time axis.

For example, in a case where the feature value calculation unit 11 calculates the area of the bladder region BR1 or BR2 in the ultrasound images U1 or U2 of the plurality of frames as the feature value, as shown in Fig. 24, the scanning success/failure determination unit 12 detects minimum values P6 and P7 smaller than a given threshold value H4 and detects a maximum value MP1 greater than a given threshold value H5 in the area of the bladder region BR1 or BR2. The scanning success/failure determination unit 12 acknowledges that the distribution of the areas of the bladder region BR1 or BR2 in the ultrasound images U1 or U2 of the plurality of frames has symmetry in the time axis direction in a case where a duration of a section T9 between a position on the time axis indicating the minimum value P6 and a position on the time axis indicating the maximum value MP1 is equal to a duration of a section T10 between a position on the time axis indicating the maximum value MP1 and a position on the time axis indicating the minimum value P7, and acknowledges that the distribution of the areas of the bladder region BR1 or BR2 in the ultrasound images U1 or U2 of the plurality of frames is asymmetrical in the time axis direction in a case where the duration of the section T9 is different from the duration of the section T10.

Here, the state in which the duration of the section T9 is equal to the duration of the section T10 means that the duration of the section T9 substantially coincides with the duration of the section T10. That is, in a case where the difference between the duration of the section T9 and the duration of the section T10 is within a given range, this means that the duration of the section T9 is equal to the duration of the section T10. In a case where the difference between the duration of the section T9 and the duration of the section T10 is outside the given range, this means that the duration of the section T9 is different from the duration of the section T10.

In an example shown in Fig. 24, the duration of the section T9 is equal to the duration of the section T10, and thus, the scanning success/failure determination unit 12 acknowledges that the distribution of the areas of the bladder region BR1 or BR2 in the ultrasound images U1 or U2 of the plurality of frames has symmetry in the time axis direction. In a case where the area of the bladder region BR1 or BR2 in the ultrasound images U1 or U2 of the plurality of frames is continuous with respect to the time axis, the scanning success/failure determination unit 12 determines that the scanning of the bladder B of the subject S with the ultrasound beam is successful.

In this case, the maximum diameter of the bladder region BR1 or BR2 is calculated from the ultrasound images U1 or U2 of the plurality of frames that are acquired by the image acquisition unit 8 in a state in which the ultrasound probe 2 is positioned directly above the center C of the bladder B of the subject S and in which the bladder B of the subject S is clearly visualized, and the volume of the bladder B of the subject S is measured as the urine volume in the bladder B based on the measured maximum diameter of the bladder region BR1 or BR2. Thus, the measurement accuracy of the urine volume is improved.

Even in a case where the distribution of the area of the bladder region BR1 or BR2 in the ultrasound images U1 or U2 of the plurality of frames is acknowledged to have symmetry in the time axis direction, for example, in a case where there is a change point where the area of the bladder region BR1 or BR2 in the ultrasound images U1 or U2 of the plurality of frames changes extremely, the scanning success/failure determination unit 12 determines that the scanning with the ultrasound beam fails.

As shown in Fig. 25, in a case where the ultrasound probe 2 is disposed at a position deviating from directly above the center of the bladder B of the subject S, and the ultrasound probe is tilted at a constant speed over the given angle range A, the distribution of the areas of the bladder region BR1 or BR2 calculated as the feature value by the feature value calculation unit 11 in the ultrasound images U1 and U2 of the plurality of frames is asymmetrical in the time axis direction as shown in Fig. 26.

In an example shown in Fig. 26, a duration of a section T11 between a position on the time axis indicating minimum value P8 of the area of the bladder region BR1 or BR2 smaller than the given threshold value H4 and a position on the time axis indicating a maximum value MP2 of the area of the bladder region BR1 or BR2 greater than the given threshold value H5 is different from a duration of a section T12 between the position on the time axis indicating the maximum value MP2 and a position on the time axis indicating a minimum value P9 of the area of the bladder region BR1 or BR2 smaller than the given threshold value H4. For this reason, the scanning success/failure determination unit 12 does not acknowledge that the distribution of the areas of the bladder region BR1 or BR2 in the ultrasound images U1 or U2 of the plurality of frames has symmetry in the time axis direction, and determines that the scanning of the bladder B of the subject S with the ultrasound beam fails.

From the above description, with the ultrasound diagnostic apparatus according to Embodiment 3 of the invention, the scanning success/failure determination unit 12 determines whether or not the scanning of the bladder B of the subject S with the ultrasound beam is successful further in consideration of the symmetry of the distribution of the feature values in the ultrasound images U1 or U2 of the plurality of frames in the time axis direction. Thus, it is possible to further improve the measurement accuracy of the urine volume in the bladder B of the subject S.

### Embodiment 4

In Embodiment 3, although the scanning success/failure determination unit 12 determines that the ultrasound probe 2 is positioned directly above the center C of the bladder B of the subject S in a case where the distribution of the feature values with respect to the time axis is acknowledged to have symmetry in the time axis direction, and determines that the ultrasound probe 2 is positioned at a position deviating from directly above the center C of the bladder B of the subject S in a case where the symmetry of the distribution of the feature values with respect to the time axis is not acknowledged, a method of determining whether or not the ultrasound probe 2 is positioned directly above the center C of the bladder B of the subject S is not limited thereto.

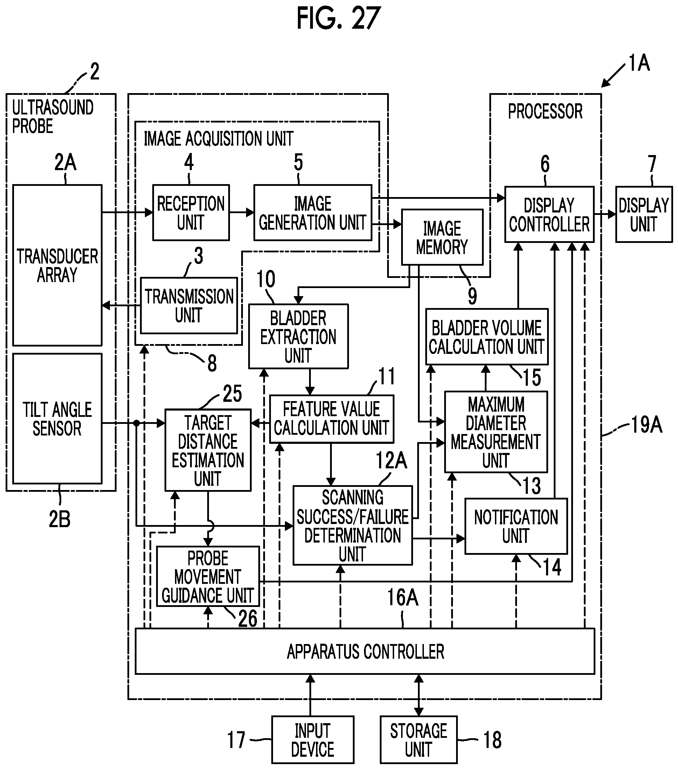

Fig. 27 shows the configuration of an ultrasound diagnostic apparatus 1A according to Embodiment 4 of the invention. The ultrasound diagnostic apparatus 1A is different from the ultrasound diagnostic apparatus 1 of Embodiment 1 shown in Fig. 1 in that a scanning success/failure determination unit 12A is provided instead of the scanning success/failure determination unit 12, an apparatus controller 16A is provided instead of the apparatus controller 16, and a tilt angle sensor 2B, a target distance estimation unit 25, and a probe movement guidance unit 26 are newly added.

In the ultrasound diagnostic apparatus 1A, the tilt angle sensor 2B is incorporated in the ultrasound probe 2, and the scanning success/failure determination unit 12A and the target distance estimation unit 25 are connected to the tilt angle sensor 2B. The feature value calculation unit 11 and the probe movement guidance unit 26 are connected to the target distance estimation unit 25. The display controller 6 is connected to the probe movement guidance unit 26.

The display controller 6, the image acquisition unit 8, the bladder extraction unit 10, the feature value calculation unit 11, the scanning success/failure determination unit 12A, the maximum diameter measurement unit 13, the notification unit 14, the bladder volume calculation unit 15, the apparatus controller 16A, the target distance estimation unit 25, and the probe movement guidance unit 26 configure a processor 19A.

The tilt angle sensor 2B measures the tilt angle W of the ultrasound probe 2 shown in Figs. 4 and 5. For example, the tilt angle sensor 2B includes a so-called gyro sensor, an acceleration sensor, a magnetic sensor, and the like, and converts electrical signals obtained from the gyro sensor, the acceleration sensor, the magnetic sensor, and the like into the tilt angle W of the ultrasound probe 2 using a known calculation method or the like. Here, it is assumed that the tilt angle W of the ultrasound probe 2 indicates 0 degrees in the ultrasound probe 2 in a state in which a direction normal to the transducer array 2A is directed in a direction perpendicular to the body surface of the subject S, and has a greater value as the ultrasound probe 2 is tilted from the state.

The tilt angle sensor 2B is incorporated in the ultrasound probe 2, but may be mounted on the ultrasound probe 2 instead of being incorporated in the ultrasound probe 2.

Here, as shown in Fig. 4, in a case where the ultrasound probe 2 is positioned directly above the center C of the bladder B of the subject S, the feature value, such as the area or the diameter of the bladder region BR1 or BR2, calculated by the feature value calculation unit 11 is maximized in a state in which the direction normal to the transducer array 2A at the center of the transducer array 2A of the ultrasound probe 2 is directed in the direction perpendicular to the body surface of the subject S. For this reason, for example, at a time at which the tilt angle of the ultrasound probe 2 measured by the tilt angle sensor 2B is 0 degrees, determination can be made whether or not the ultrasound probe 2 is positioned directly above the center C of the bladder B of the subject S depending on whether or not the feature value, such as the area or the diameter of the bladder region BR1 or BR2, is maximized.

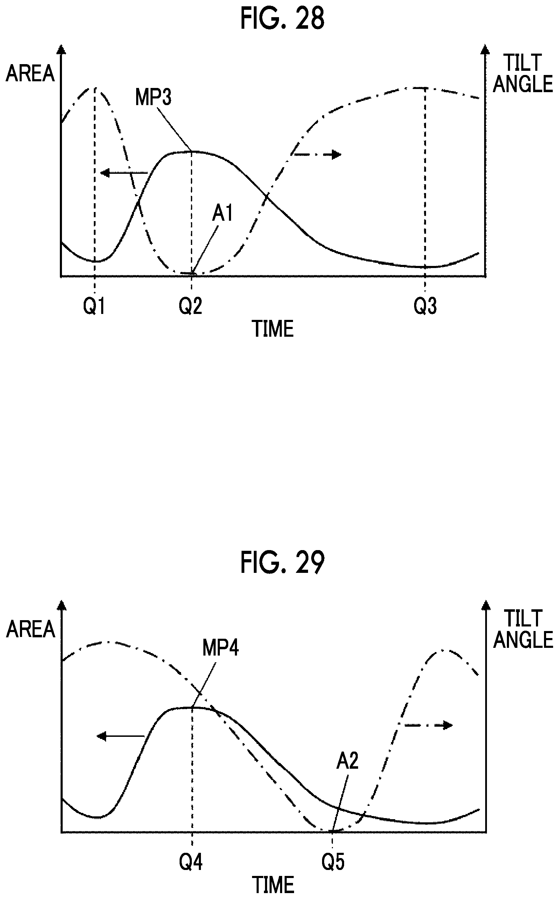

Accordingly, the scanning success/failure determination unit 12A determines whether or not the scanning of the bladder B of the subject S with the ultrasound beam is successful in consideration of a relationship between the tilt angle W of the ultrasound probe 2 measured by the tilt angle sensor 2B and the distribution of the feature values calculated by the feature value calculation unit 11 in the ultrasound images U1 or U2 of the plurality of frames with respect to the time axis. For example, in a case where the feature value calculation unit 11 calculates the area of the bladder region BR1 or BR2 in the ultrasound images U1 or U2 of the plurality of frames as the feature value, the scanning success/failure determination unit 12A determines that the scanning with the ultrasound beam is successful in a case where the difference value of the areas of the bladder region BR1 or BR2 between any frames continuous in time series is smaller than the given threshold value, and as shown in Fig. 28, a position where the area of the bladder region BR1 or BR2 indicates the maximum value is acknowledged to coincide with a position where the tilt angle W measured by the tilt angle sensor 2B indicates the minimum value, on the time axis.

In an example shown in Fig. 28, at a time Q1 and a time Q3, the area of the bladder region BR1 or BR2 has a minimum value and the tilt angle W of the ultrasound probe 2 has a maximum value. At a time Q2, the area of the bladder region BR1 or BR2 has a maximum value MP3 and the tilt angle W of the ultrasound probe 2 has a minimum value A1.

Here, the state in which the position where the area of the bladder region BR1 or BR2 indicates the maximum value coincides with the position where the tilt angle W measured by the tilt angle sensor 2B indicates the minimum value means that the position where the area of the bladder region BR1 or BR2 indicates the maximum value substantially coincides with the position where the tilt angle W measured by the tilt angle sensor 2B indicates the minimum value. That is, in a case where a time interval corresponding to a section between the position where the area of the bladder region BR1 or BR2 indicates the maximum value and the position where the tilt angle W measured by the tilt angle sensor 2B indicates the minimum value is within a given range, this means that the position where the area of the bladder region BR1 or BR2 indicates the maximum value coincides with the position where the tilt angle W measured by the tilt angle sensor 2B indicates the minimum value. In a case where a time interval corresponding to a section between the position where the area of the bladder region BR1 or BR2 indicates the maximum value and the position where the tilt angle W measured by the tilt angle sensor 2B indicates the minimum value is outside the given range, this means that the position where the area of the bladder region BR1 or BR2 indicates the maximum value does not coincide with the position where the tilt angle W measured by the tilt angle sensor 2B indicates the minimum value.

For example, while the difference value of the areas of the bladder region BR1 or BR2 in at least one frame continuous in time series is equal to or greater than the given threshold value, as shown in Fig. 29, in a case where the position where the area of the bladder region BR1 or BR2 indicates the maximum value is not acknowledged to coincide with the position where the tilt angle W measured by the tilt angle sensor 2B indicates the minimum value, on the time axis, the scanning success/failure determination unit 12A determines that the scanning with the ultrasound beam fails.

In an example shown in Fig. 29, a time Q4 at which the area of the bladder region BR1 or BR2 has a maximum value MP4 does not coincide with a time Q at which the tilt angle W of the ultrasound probe 2 has a minimum value A2.

The target distance estimation unit 25 estimates a distance between the center C of the bladder B of the subject S and the contact position of the ultrasound probe 2 with the subject S in a direction along the body surface of the subject S based on an ultrasound image of a frame representing a tomographic plane passing through the center C of the bladder B of the subject S among the ultrasound images U1 or U2 of the plurality of frames acquired by the image acquisition unit 8 and the tilt angle W of the ultrasound probe 2 measured by the tilt angle sensor 2B.

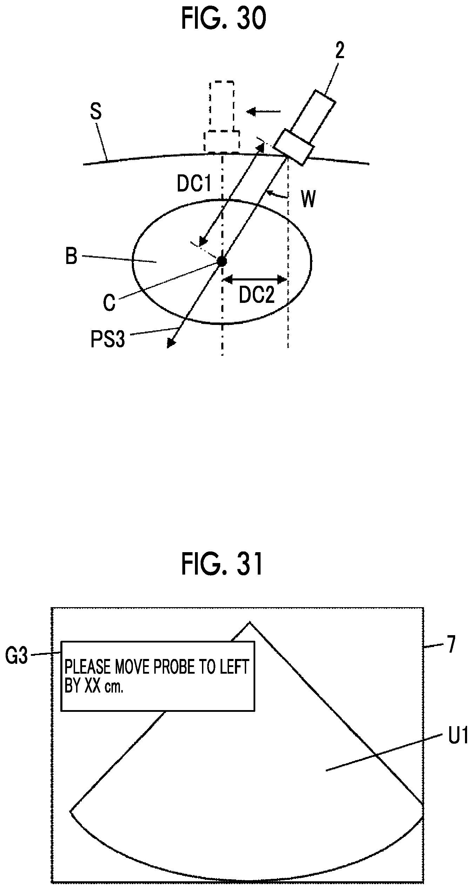

For example, as shown in Fig. 30, the target distance estimation unit 25 selects a frame where the area or the diameter of the bladder region BR1 in the ultrasound image U1 is maximized, among the ultrasound images U1 of the plurality of frames as a frame where a scanning plane PS3 from the ultrasound probe 2 passes through the center C of the bladder B of the subject S, that is, an ultrasound image representing the tomographic plane passing through the center C of the bladder B of the subject S. The target distance estimation unit 25 measures a distance DC1 between the contact position of the ultrasound probe 2 with the subject S and the center C of the bladder B of the subject S from the ultrasound image of the selected frame.

The target distance estimation unit 25 can estimate a distance DC2 between the center C of the bladder B of the subject S and the contact position of the ultrasound probe 2 with the subject S in the direction along the body surface of the subject S, that is, the distance DC2 between the contact position of the ultrasound probe 2 with the subject S and the position directly above the bladder B of the subject S by calculating DC1 × sin(W) using the measured distance DC1 and the tilt angle W of the ultrasound probe 2 measured by the tilt angle sensor 2B.

The probe movement guidance unit 26 guides the user to position the ultrasound probe 2 directly above the center C of the bladder B of the subject S by moving the ultrasound probe 2 along the body surface of the subject S by the distance DC2 estimated by the target distance estimation unit 25. For example, as shown in Fig. 31, the probe movement guidance unit 26 can display information for moving the ultrasound probe 2 along the body surface of the subject S by the distance estimated by the target distance estimation unit 25 on the display unit 7. In an example shown in Fig. 31, a guide panel G3 including text data "Please move probe to left by XX cm." is displayed on the display unit 7 to be superimposed on the ultrasound image U1.

Though not shown, in a case where the ultrasound diagnostic apparatus 1A comprises a speaker, the probe movement guidance unit 26 can perform guidance to the user by voice through the speaker.

From the above description, with the ultrasound diagnostic apparatus 1A according to Embodiment 4, determination is made whether or not the scanning of the bladder B of the subject S with the ultrasound beam is successful in consideration of whether or not a position where a feature value calculated by the feature value calculation unit 11 indicates an extreme value coincides with a position where the tilt angle W measured by the tilt angle sensor 2B indicates an extreme value, on the time axis. Thus, it is possible to further improve the measurement accuracy of the urine volume in the bladder B of the subject S.

In a case where the position where the feature value calculated by the feature value calculation unit 11 indicates the extreme value does not coincide with the position where the tilt angle W measured by the tilt angle sensor 2B indicates the extreme value, on the time axis, the target distance estimation unit 25 estimates the distance DC2 between the contact position of the ultrasound probe 2 with the subject S and the position directly above the bladder B of the subject S, and the probe movement guidance unit 26 guides the user to move the ultrasound probe 2 along the body surface of the subject S by the estimated distance DC2 based on the estimated distance DC2. Thus, the user easily disposes the ultrasound probe 2 at the position directly above the bladder B of the subject S, whereby it is possible to further improve the measurement accuracy of the urine volume in the bladder B of the subject S.

### Embodiment 5

Although the ultrasound diagnostic apparatus 1 according to Embodiment 1 has a configuration in which the ultrasound probe 2, the display unit 7, and the input device 17 are connected directly to the processor 19, for example, the ultrasound probe 2, the display unit 7, and the input device 17 may be connected directly through a network.

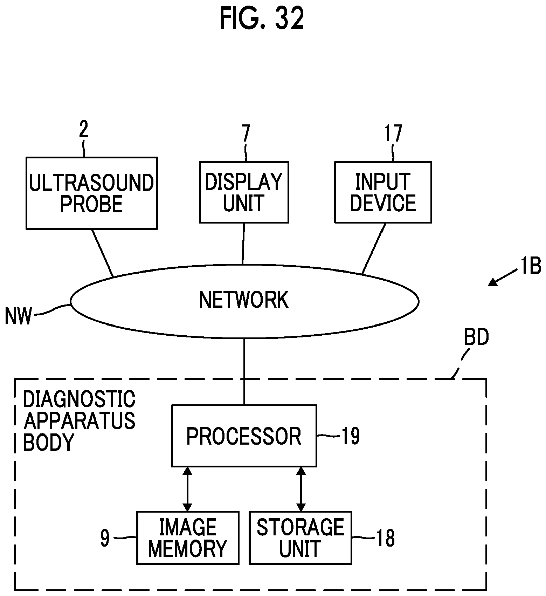

As shown in Fig. 32, an ultrasound diagnostic apparatus 1B according to Embodiment 5 has a configuration in which the ultrasound probe 2, the display unit 7, and the input device 17 are connected to a diagnostic apparatus body BD through a network NW. The diagnostic apparatus body BD has a configuration in which the ultrasound probe 2, the display unit 7, and the input device 17 are removed in the ultrasound diagnostic apparatus 1 of Embodiment 1 shown in Fig. 1.

Here, in a case where the ultrasound beam is transmitted from the transducer array 2A of the ultrasound probe 2 into the subject S in a case where the ultrasound probe 2 is pressed to the subject S by the user, the transducer array 2A receives an ultrasound echo reflected inside the subject S and generates a reception signal. The ultrasound probe 2 transmits the generated reception signal to the diagnostic apparatus body BD through the network NW. The image acquisition unit 8 of the processor 19 of the diagnostic apparatus body BD receives the reception signal transmitted from the ultrasound probe 2 in this manner through the network NW, and the image acquisition unit 8 generates an ultrasound image U1 or U2 based on the reception signal.

The ultrasound image U1 or U2 generated by the image acquisition unit 8 is sent to the display controller 6 and the image memory 9. The display controller 6 executes predetermined processing on the ultrasound image U1 or U2 received from the image acquisition unit 8 and further transmits the ultrasound image U1 or U2 subjected to the predetermined processing to the display unit 7 through the network NW. In this manner, the display unit 7 receives and displays the ultrasound image U1 or U2 transmitted from the display controller 6 of the processor 19 of the diagnostic apparatus body BD through the network NW.

The bladder extraction unit 10 of the processor 19 extracts a bladder region BR1 or BR2 representing the bladder B of the subject S from the ultrasound images of a plurality of frames stored in the image memory 9.

The feature value calculation unit 11 calculates a feature value, such as the area of the bladder region BR1 or BR2, representing the feature of the bladder region BR1 or BR2 extracted by the bladder extraction unit 10 in the ultrasound images U1 or U2 of the plurality of frames.

The scanning success/failure determination unit 12 determines whether or not scanning of the bladder B of the subject S with an ultrasound beam is successful based on the feature value calculated by the feature value calculation unit 11 in the ultrasound images U1 and U2 of the plurality of frames.

The maximum diameter measurement unit 13 measures the maximum diameter of the bladder region BR1 or BR2 from the ultrasound images U1 or U2 of the plurality of frames stored in the image memory 9 in a case where the scanning success/failure determination unit 12 determines that the scanning with the ultrasound beam is successful.

The bladder volume calculation unit 15 calculates the volume of the bladder B of the subject S as the urine volume of the bladder B based on the maximum diameter of the bladder region BR1 or BR2 measured by the maximum diameter measurement unit 13. Information representing the urine volume in the bladder B calculated in this manner is sent to the display controller 6, and is further transmitted from the display controller 6 to the display unit 7 through the network NW. The display unit 7 receives and displays information representing the urine volume in the bladder B of the subject S.

As described above, with the ultrasound diagnostic apparatus 1B according to Embodiment 5 of the invention, even in a case where the ultrasound probe 2, the display unit 7, the input device 17, and the diagnostic apparatus body BD are connected through the network NW, similarly to the ultrasound diagnostic apparatus 1 of Embodiment 1, the scanning success/failure determination unit 12 determines whether or not the scanning of the bladder B of the subject S with the ultrasound beam is successful. Thus, the urine volume in the bladder B of the subject S is measured using only the ultrasound image U1 or U2 of the frames where the scanning with the ultrasound beam is successful, and the measurement accuracy of the urine volume in the bladder B can be improved.

The ultrasound probe 2, the display unit 7, and the input device 17 are connected to the diagnostic apparatus body BD through the network, and thus, the diagnostic apparatus body BD can be used as a so-called remote server. With this, the user can perform ultrasonography of the subject S by preparing only the ultrasound probe 2, the display unit 7, and the input device 17 at hand, and thus, convenience in ultrasonography can be improved.

For example, in a case where a portable thin computer, called a tablet, is used as the display unit 7 and the input device 17, it is possible to allow the user to more easily perform ultrasonography of the subject S, and to further improve convenience in ultrasonography.

Although the ultrasound probe 2, the display unit 7, and the input device 17 are connected to the diagnostic apparatus body BD through the network NW, the ultrasound probe 2, the display unit 7, the input device 17, and the diagnostic apparatus body BD may be connected to the network NW in a wired manner or a wireless manner.

Although an example where the aspect of Embodiment 5 is applied to Embodiment 1 has been described, the aspect of Embodiment 5 can be similarly applied to Embodiments 2, 3, and 4.

### Explanation of References

1: ultrasound diagnostic apparatus
2: ultrasound probe
2A: transducer array
3: transmission unit
4: reception unit
5: image generation unit
6: display controller
7: display unit
8: image acquisition unit
9: image memory
10: bladder extraction unit
11: feature value calculation unit
12: scanning success/failure determination unit
13: maximum diameter measurement unit
14: notification unit
15: bladder volume calculation unit
16: apparatus controller
17: input device
18: storage unit
19: processor
20: amplification unit
21: AD conversion unit
22: signal processing unit
23: DSC
24: image processing unit
A: angle range
A1, A2, P1, P2, P3, P4, P5, P6, P7, P8, P9: minimum value
B: bladder
BR1, BR2, BR3, BR4: bladder region
D1: lateral direction
D2: longitudinal direction
C: center
CP1, CP2, CP3, CP4, CP5, CP6, CP7, CP8, CP9: change point
DC1, DC2: distance
E: ellipsoid
G1, G2, G3: guide panel
H1, H2, H3, H4, H5: threshold value
LX, LY, LZ: maximum diameter
ML1, ML2, ML3: measurement line
MP1, MP2, MP3, MP4: maximum value
PA, PB: minimum value
PP1: first contact position
PP2: second contact position
PS1, PS2, PS3: scanning plane
Q1, Q2, Q3, Q4, Q5: time
R: rotation axis
RA, RB: region
S: subject
T1, T2, T3, T4, T5, T6, T7, T8, T9, T10, T11, T12: section
U1, U2, U3, U4: ultrasound image
W: tilt angle.

## Claims

1. An ultrasound diagnostic apparatus (1) comprising:
an ultrasound probe (2) configured to be brought into contact with a subject and scan the subject with an ultrasound beam;
an image acquisition unit (8) configured to acquire ultrasound images of a plurality of frames corresponding to a plurality of different tomographic planes in the subject using the ultrasound probe;
an image memory (9) configured to keep the ultrasound images of the plurality of frames acquired by the image acquisition unit;
a bladder extraction unit (10) configured to extract a bladder region from each of the ultrasound images of the plurality of frames; **characterized in that** the ultrasound diagnostic apparatus further comprises
a feature value calculation unit (11) configured to calculate a feature value regarding the bladder region extracted by the bladder extraction unit in each of the ultrasound images of the plurality of frames; and
a scanning success/failure determination unit (12) configured to analyse change in the feature value calculated by the feature value calculation unit between frames continuous in time series and configured to determine whether or not scanning of a bladder of the subject with the ultrasound beam is successful based on an analysis result.

2. The ultrasound diagnostic apparatus according to claim 1,
wherein the ultrasound images of the plurality of frames are ultrasound images that are acquired by the image acquisition unit while changing a tilt angle of the ultrasound probe over a given angle range with respect to the subject while a contact position of the ultrasound probe with the subject is fixed.

3. The ultrasound diagnostic apparatus according to claim 2, further comprising:
a maximum diameter measurement unit (13) configured to measure a maximum diameter of the bladder region using the ultrasound images of the plurality of frames in a case where the scanning success/failure determination unit determines that the scanning with the ultrasound beam is successful.

4. The ultrasound diagnostic apparatus according to claim 2,
wherein the ultrasound images of the plurality of frames are ultrasound images that are acquired by the image acquisition unit in a case where a plurality of times of scanning of the bladder of the subject with the ultrasound beam are performed.

5. The ultrasound diagnostic apparatus according to claim 4,
wherein the scanning success/failure determination unit (12) is configured to determine whether or not each of the plurality of times of scanning with the ultrasound beam is successful.

6. The ultrasound diagnostic apparatus according to claim 5, further comprising:
a maximum diameter measurement unit (13) configured to measure a maximum diameter of the bladder region using only ultrasound images of a plurality of frames acquired by scanning with the ultrasound beam determined to be successful by the scanning success/failure determination unit among the plurality of times of scanning with the ultrasound beam.

7. The ultrasound diagnostic apparatus according to any one of claims 2 to 6,
wherein the feature value is an area or a diameter of the bladder region extracted by the bladder extraction unit in the ultrasound image.

8. The ultrasound diagnostic apparatus according to claim 7,
wherein the scanning success/failure determination unit (12) is configured to determine whether or not the scanning of the bladder of the subject with the ultrasound beam is successful by analyzing continuity of change in the feature value between frames continuous in time series.

9. The ultrasound diagnostic apparatus according to claim 8,
wherein the scanning success/failure determination unit (12) is configured to determine that the scanning with the ultrasound beam is successful in a case where a difference value of the feature values between any frames continuous in time series in the ultrasound images of the plurality of frames is smaller than a given threshold value, and is configured to determine that the scanning with the ultrasound beam fails in a case where a difference value of the feature values in at least one frame continuous in time series in the ultrasound images of the plurality of frames is equal to or greater than the threshold value.

10. The ultrasound diagnostic apparatus according to claim 8,
wherein the ultrasound images of the plurality of frames are ultrasound images that are acquired by the image acquisition unit while changing the tilt angle of the ultrasound probe at a constant speed while the contact position of the ultrasound probe with the subject is fixed, and
the scanning success/failure determination unit (12) is configured to determine that the scanning with the ultrasound beam is successful in a case where a difference value of the feature values between any frames continuous in time series in the ultrasound images of the plurality of frames is smaller than a given threshold value, and symmetry of a distribution of the feature values with respect to a time axis is acknowledged, and is configured to determine that the scanning with the ultrasound beam fails in a case where a difference value of the feature values in at least one frame continuous in time series in the ultrasound images of the plurality of frames is equal to or greater than the threshold value or in a case where the symmetry of the distribution of the feature values with respect to the time axis is not acknowledged.

11. The ultrasound diagnostic apparatus according to claim 10,
wherein the scanning success/failure determination unit (12) is configured to determine that the symmetry of the distribution of the feature values with respect to the time axis is acknowledged in a case where a position where the feature value is maximized is positioned in a middle point of a pair of positions where the feature value is minimized, on the time axis, and is configured to determine that the symmetry of the distribution of the feature values with respect to the time axis is acknowledged in a case where the position where the feature value is maximized deviates from the middle point of the pair of positions where the feature value is minimized, on the time axis.

12. The ultrasound diagnostic apparatus according to claim 8, further comprising: a tilt angle sensor configured to measure the tilt angle of the ultrasound probe,
wherein the scanning success/failure determination unit (12) is configured to determine that the scanning with the ultrasound beam is successful in a case where a difference value of the feature values between any frames continuous in time series in the ultrasound images of the plurality of frames is smaller than a given threshold value, and a position where the feature value indicates an extreme value is acknowledged to coincide with a position where the tilt angle measured by the tilt angle sensor indicates an extreme value, on a time axis, and is configured to determine that the scanning with the ultrasound beam fails in a case where a difference value of the feature values in at least one frame continuous in time series is equal to or greater than the threshold value or the position where the feature value with respect to the time axis indicates the extreme value is not acknowledged to coincide with the position where the tilt angle measured by the tilt angle sensor indicates the extreme value.

13. The ultrasound diagnostic apparatus according to claim 12, further comprising:
a target distance estimation unit configured to estimate a distance between a center of the bladder of the subject and the contact position of the ultrasound probe with the subject in a direction along a body surface of the subject based on an ultrasound image of a frame representing a tomographic plane passing the center of the bladder of the subject among the ultrasound images of the plurality of frames acquired by the image acquisition unit and the tilt angle of the ultrasound probe measured by the tilt angle sensor; and
a probe movement guidance unit configured to guide a user to position the ultrasound probe directly above the center of the bladder of the subject by moving the ultrasound probe along the body surface of the subject by the distance estimated by the target distance estimation unit.

14. The ultrasound diagnostic apparatus according to any one of claims 2 to 6,
wherein the feature value is a position of the bladder region extracted by the bladder extraction unit in the ultrasound image.

15. The ultrasound diagnostic apparatus according to claim 14,
wherein the feature value is a position of a center of gravity of the bladder region extracted by the bladder extraction unit in the ultrasound image, and
the scanning success/failure determination unit (12) is configured to determine that the scanning with the ultrasound beam is successful in a case where a distance between the centers of gravity of the bladder regions in frames continuous in time series is smaller than a given threshold value, and is configured to determine that the scanning with the ultrasound beam fails in a case where the distance between the centers of gravity of the bladder regions in the frames continuous in time series is equal to or greater than the given threshold value.

16. The ultrasound diagnostic apparatus according to claim 14,
wherein the scanning success/failure determination unit (12) is configured to determine that the scanning with the ultrasound beam is successful in a case where a ratio of an area of a region where the bladder regions overlap each other to an area of a region occupied by at least one of the bladder regions in the frames continuous in time series is equal to or greater than a given threshold value, and is configured to determine that the scanning of the ultrasound beam fails in a case where the ratio of the area of the region where the bladder regions overlap each other to the area of the region occupied by at least one of the bladder regions in the frames continuous in time series is smaller than the given threshold value.

17. The ultrasound diagnostic apparatus according to any one of claims 1 to 16, further comprising:
a notification unit (14) that, in a case where the scanning success/failure determination unit determines that the scanning of the ultrasound beam fails, is configured to notify a user that the scanning of the ultrasound beam fails.

18. The ultrasound diagnostic apparatus according to any one of claims 1 to 17,
wherein the image acquisition unit (8) is configured to acquire a first group of ultrasound images of a plurality of frames corresponding to the plurality of tomographic planes of the bladder of the subject along a lateral direction and a second group of ultrasound images of a plurality of frames corresponding to the plurality of tomographic planes of the bladder of the subject along a longitudinal direction,
the bladder extraction unit (10) is configured to extract the bladder region from each of the first group of the ultrasound images of the plurality of frames and the second group of the ultrasound images of the plurality of frames,
the feature value calculation unit (11) is configured to calculate the feature value from each of the first group of the ultrasound images of the plurality of frames and the second group of the ultrasound images of the plurality of frames, and
the scanning success/failure determination unit (12) is configured to determine whether or not the scanning of the bladder of the subject with the ultrasound beam is successful for each of the first group of the ultrasound images of the plurality of frames and the second group of the ultrasound images of the plurality of frames.

19. The ultrasound diagnostic apparatus according to claim 18, further comprising:
a bladder volume calculation unit (15) that is configured to calculate a volume of the bladder based on a maximum diameter of the bladder region measured from the first group of the ultrasound images of the plurality of frames in the lateral direction, a maximum diameter of the bladder region measured from the second group of the ultrasound images of the plurality of frames in the longitudinal direction, and a maximum diameter of the bladder region measured from the first group of the ultrasound images of the plurality of frames or the second group of the ultrasound images of the plurality of frames in a depth direction.

20. A method of controlling an ultrasound diagnostic apparatus, the method comprising:
acquiring ultrasound images of a plurality of frames corresponding to a plurality of different tomographic planes in a subject using an ultrasound probe that is brought into contact with the subject and scans the subject with an ultrasound beam;
keeping the acquired ultrasound images of the plurality of frames;
extracting a bladder region from each of the ultrasound images of the plurality of frames; **characterized in that** the method further comprises
calculating a feature value regarding the bladder region extracted in each of the ultrasound images of the plurality of frames; and
analyzing change in the calculated feature value between frames continuous in time series and determining whether or not scanning of a bladder of the subject with the ultrasound beam is successful based on an analysis result.

## Patentansprüche

1. Ein Ultraschall-Diagnosegerät (1), das Folgendes umfasst:
eine Ultraschallsonde (2), die so konfiguriert ist, dass sie mit einem Subjekt in Kontakt gebracht wird und das Subjekt mit einem Ultraschallstrahl abtastet;
eine Bilderfassungseinheit (8), die so konfiguriert ist, dass sie unter Verwendung der Ultraschallsonde Ultraschallbilder einer Vielzahl von Einzelbildern erfasst, die einer Vielzahl von verschiedenen tomographischen Ebenen in dem Subjekt entsprechen;
einen Bildspeicher (9), der so konfiguriert ist, dass er die von der Bilderfassungseinheit erfassten Ultraschallbilder der Vielzahl von Bildern speichert;
eine Blasenextraktionseinheit (10), die so konfiguriert ist, dass sie einen Blasenbereich aus jedem der Ultraschallbilder aus der Vielzahl der Bilder extrahiert;
**dadurch gekennzeichnet, dass** die Ultraschall-Diagnosevorrichtung ferner eine Merkmalswert-Berechnungseinheit (11) umfasst, die so konfiguriert ist, dass sie einen Merkmalswert in Bezug auf den durch die Blasenextraktionseinheit extrahierten Blasenbereich in jedem der Ultraschallbilder der Vielzahl von Rahmen berechnet; und
eine Abtasterfolgs/Fehlschlag bzw. Misserfolg -Bestimmungseinheit (12), die so konfiguriert ist, dass sie die Änderung des von der Merkmalswert-Berechnungseinheit berechneten Merkmalswerts zwischen zeitlich fortlaufenden Rahmen analysiert, und die so konfiguriert ist, dass sie auf der Grundlage eines Analyseergebnisses bestimmt, ob das Abtasten einer Blase des Subjekts mit dem Ultraschallstrahl erfolgreich ist oder nicht.

2. Ultraschall-Diagnosegerät nach Anspruch 1,
wobei die Ultraschallbilder der mehreren Bilder Ultraschallbilder sind, die von der Bilderfassungseinheit erfasst werden, während ein Neigungswinkel der Ultraschallsonde über einen gegebenen Winkelbereich in Bezug auf das Subjekt verändert wird, während eine Kontaktposition der Ultraschallsonde mit dem Subjekt festgelegt ist.

3. Ultraschall-Diagnosegerät nach Anspruch 2, das außerdem Folgendes umfasst:
eine Einheit (13) zur Messung des maximalen Durchmessers, die so konfiguriert ist, dass sie einen maximalen Durchmesser des Blasenbereichs unter Verwendung der Ultraschallbilder der mehreren Rahmen in einem Fall misst, in dem die Einheit zur Bestimmung des Erfolgs/Fehlschlags der Abtastung bestimmt, dass die Abtastung mit dem Ultraschallstrahl erfolgreich ist.

4. Ultraschall-Diagnosegerät nach Anspruch 2,
wobei die Ultraschallbilder der Vielzahl von Bildern Ultraschallbilder sind, die von der Bilderfassungseinheit in einem Fall erfasst werden, in dem eine Vielzahl von Abtastungen der Blase des Patienten mit dem Ultraschallstrahl durchgeführt werden.

5. Ultraschall-Diagnosegerät nach Anspruch 4,
wobei die Einheit (12) zur Bestimmung des Erfolgs oder Fehlschlags bzw. Misserfolgs der Abtastung so konfiguriert ist, dass sie bestimmt, ob jede der mehreren Abtastungen mit dem Ultraschallstrahl erfolgreich ist oder nicht.

6. Das Ultraschall-Diagnosegerät nach Anspruch 5, das ferner Folgendes umfasst:
eine Einheit (13) zur Messung des maximalen Durchmessers, die so konfiguriert ist, dass sie einen maximalen Durchmesser des Blasenbereichs misst, indem sie nur Ultraschallbilder einer Vielzahl von Rahmen verwendet, die durch Abtasten mit dem Ultraschallstrahl erfasst wurden, die von der Einheit zur Bestimmung des Erfolgs oder Misserfolgs des Abtastens unter der Vielzahl von Malen des Abtastens mit dem Ultraschallstrahl als erfolgreich bestimmt wurden.

7. Ultraschall-Diagnosegerät nach einem der Ansprüche 2 bis 6,
wobei der Merkmalswert eine Fläche oder ein Durchmesser des von der Blasenextraktionseinheit in dem Ultraschallbild extrahierten Blasenbereichs ist.

8. Ultraschall-Diagnosegerät nach Anspruch 7,
wobei die Einheit (12) zur Bestimmung des Erfolgs oder Fehlschlags der Abtastung so konfiguriert ist, dass sie bestimmt, ob die Abtastung der Blase des Subjekts mit dem Ultraschallstrahl erfolgreich ist oder nicht, indem sie die Kontinuität der Änderung des Merkmalswerts zwischen in zeitlicher Reihe bzw. Zeitreihe kontinuierlichen Bildern analysiert.

9. Ultraschall-Diagnosegerät nach Anspruch 8,
wobei die Abtasterfolgs/Fehlschlag-Bestimmungseinheit (12) konfiguriert ist, um zu bestimmen, dass das Abtasten mit dem Ultraschallstrahl in einem Fall erfolgreich ist, in dem ein Differenzwert der Merkmalswerte zwischen beliebigen Rahmen, die in zeitlicher reihe bzw. Zeitreihe in den Ultraschallbildern der Vielzahl von Rahmen fortlaufend sind, kleiner als ein gegebener Schwellenwert ist, und konfiguriert ist, um zu bestimmen, dass das Abtasten mit dem Ultraschallstrahl in einem Fall fehlschlägt, in dem ein Differenzwert der Merkmalswerte in mindestens einem Rahmen, der in der Zeitreihe in den Ultraschallbildern der Vielzahl von Rahmen fortlaufend ist, gleich oder größer als der Schwellenwert ist.

10. Ultraschall-Diagnosegerät nach Anspruch 8,
wobei die Ultraschallbilder der Vielzahl von Einzelbildern Ultraschallbilder sind, die von der Bilderfassungseinheit erfasst werden, während der Neigungswinkel der Ultraschallsonde mit einer konstanten Geschwindigkeit verändert wird, während die Kontaktposition der Ultraschallsonde mit dem Subjekt fest verbunden ist, und
die Abtast-Erfolgs/Misserfolgs-Bestimmungseinheit (12) konfiguriert ist, um zu bestimmen, dass die Abtastung mit dem Ultraschallstrahl in einem Fall erfolgreich ist, in dem ein Differenzwert der Merkmalswerte zwischen irgendwelchen Rahmen, die in Zeitreihen in den Ultraschallbildern der Vielzahl von Rahmen fortlaufend sind, kleiner als ein gegebener Schwellenwert ist, und die Symmetrie einer Verteilung der Merkmalswerte in Bezug auf eine Zeitachse bestätigt wird, und konfiguriert ist, um zu bestimmen, dass die Abtastung mit dem Ultraschallstrahl in einem Fall fehlschlägt, in dem ein Differenzwert der Merkmalswerte in mindestens einem in der Zeitreihe kontinuierlichen Rahmen in den Ultraschallbildern der Vielzahl von Rahmen gleich oder größer als der Schwellenwert ist, oder in einem Fall, in dem die Symmetrie der Verteilung der Merkmalswerte in Bezug auf die Zeitachse nicht bestätigt wird.

11. Ultraschall-Diagnosegerät nach Anspruch 10,
wobei die Abtasterfolgs/Fehlschlags-Bestimmungseinheit (12) konfiguriert ist, um zu bestimmen, dass die Symmetrie der Verteilung der Merkmalswerte in Bezug auf die Zeitachse in einem Fall anerkannt wird, in dem eine Position, in der der Merkmalswert maximiert ist, in einem Mittelpunkt eines Paares von Positionen positioniert ist, in denen der Merkmalswert minimiert ist, auf der Zeitachse positioniert ist, und konfiguriert ist, um zu bestimmen, dass die Symmetrie der Verteilung der Merkmalswerte in Bezug auf die Zeitachse in einem Fall bestätigt wird, in dem die Position, in der der Merkmalswert maximiert ist, von dem mittleren Punkt des Paares von Positionen, in denen der Merkmalswert minimiert ist, auf der Zeitachse abweicht.

12. Ultraschall-Diagnosegerät nach Anspruch 8, ferner umfassend:
einen Neigungswinkelsensor, der zur Messung des Neigungswinkels der Ultraschallsonde konfiguriert ist,
wobei die Abtasterfolgs/Fehlschlag-Bestimmungseinheit (12) konfiguriert ist, um zu bestimmen, dass das Abtasten mit dem Ultraschallstrahl in einem Fall erfolgreich ist, in dem ein Differenzwert der Merkmalswerte zwischen irgendwelchen in der Zeitreihe kontinuierlichen Rahmen in den Ultraschallbildern der Vielzahl von Rahmen kleiner als ein gegebener Schwellenwert ist, und eine Position, in der der Merkmalswert einen Extremwert anzeigt, als mit einer Position übereinstimmend erkannt wird, in der der von dem Neigungswinkelsensor gemessene Neigungswinkel einen Extremwert anzeigt, auf einer Zeitachse anzeigt, und konfiguriert ist, um zu bestimmen, dass das Abtasten mit dem Ultraschallstrahl in einem Fall fehlschlägt, in dem ein Differenzwert der Merkmalswerte in mindestens einem in der Zeitreihe fortlaufenden Rahmen gleich oder größer als der Schwellenwert ist oder die Position, in der der Merkmalswert in Bezug auf die Zeitachse den Extremwert anzeigt, nicht als mit der Position zusammenfallend erkannt wird, in der der durch den Neigungswinkelsensor gemessene Neigungswinkel den Extremwert anzeigt.

13. Ultraschall-Diagnosegerät nach Anspruch 12, das ferner Folgendes umfasst:
eine Zielabstandsschätzungseinheit, die so konfiguriert ist, dass sie einen Abstand zwischen einem Zentrum der Blase des Subjekts und der Kontaktposition der Ultraschallsonde mit dem Subjekt in einer Richtung entlang einer Körperoberfläche des Subjekts auf der Grundlage eines Ultraschallbildes eines Rahmens, der eine tomographische Ebene darstellt, die durch das Zentrum der Blase des Subjekts unter den Ultraschallbildern der Vielzahl von Rahmen hindurchgeht, die von der Bilderfassungseinheit erfasst werden, und des Neigungswinkels der Ultraschallsonde, der von dem Neigungswinkelsensor gemessen wird, schätzt; und
eine Sondenbewegungsführungseinheit, die so konfiguriert ist, dass sie einen Benutzer dazu anleitet, die Ultraschallsonde direkt über dem Zentrum der Blase des Subjekts zu positionieren, indem sie die Ultraschallsonde entlang der Körperoberfläche des Subjekts durch die von der Zielentfernungsschätzungseinheit geschätzte Entfernung bewegt.

14. Ultraschall-Diagnosegerät nach einem der Ansprüche 2 bis 6,
wobei der Merkmalswert eine Position des von der Blasenextraktionseinheit im Ultraschallbild extrahierten Blasenbereichs ist.

15. Ultraschall-Diagnosegerät nach Anspruch 14,
wobei der Merkmalswert eine Position eines Schwerpunkts des Blasenbereichs ist, der von der Blasenextraktionseinheit in dem Ultraschallbild extrahiert wird, und
die Abtast-Erfolgs/Misserfolgs-Bestimmungseinheit (12) so konfiguriert ist, dass sie bestimmt, dass die Abtastung mit dem Ultraschallstrahl erfolgreich ist, wenn ein Abstand zwischen den Schwerpunkten der Blasenbereiche in zeitlich fortlaufenden Rahmen kleiner als ein gegebener Schwellenwert ist, und so konfiguriert ist, dass sie bestimmt, dass die Abtastung mit dem Ultraschallstrahl misslingt bzw. fehlschlägt, wenn der Abstand zwischen den Schwerpunkten der Blasenbereiche in den zeitlich fortlaufenden Rahmen gleich dem oder größer als der gegebene Schwellenwert ist.

16. Ultraschall-Diagnosegerät nach Anspruch 14,
wobei die Abtast-Erfolgs/Fehlschlag-Bestimmungseinheit (12) konfiguriert ist, um zu bestimmen, dass die Abtastung mit dem Ultraschallstrahl in einem Fall erfolgreich ist, in dem ein Verhältnis einer Fläche eines Bereichs, in dem sich die Blasenbereiche gegenseitig überlappen, zu einer Fläche eines Bereichs, der von mindestens einem der Blasenbereiche in den zeitlich fortlaufenden Einzelbildern belegt ist, gleich oder größer als ein gegebener Schwellenwert ist, und konfiguriert ist, um zu bestimmen, dass die Abtastung des Ultraschallstrahls in einem Fall fehlschlägt, in dem das Verhältnis der Fläche des Bereichs, in dem sich die Blasenbereiche überlappen, zu der Fläche des Bereichs, der von mindestens einem der Blasenbereiche in den zeitlich fortlaufenden Bildern eingenommen wird, kleiner als der gegebene Schwellenwert ist.

17. Das Ultraschall-Diagnosegerät nach einem der Ansprüche 1 bis 16, ferner umfassend:
eine Benachrichtigungseinheit (14), die in einem Fall, in dem die Abtasterfolgs/Fehlschlags-Bestimmungseinheit bestimmt, dass das Abtasten des Ultraschallstrahls fehlschlägt, konfiguriert ist, um einen Benutzer zu benachrichtigen, dass das Abtasten des Ultraschallstrahls fehlschlägt.

18. Ultraschall-Diagnosegerät nach einem der Ansprüche 1 bis 17,
wobei die Bilderfassungseinheit (8) so konfiguriert ist, dass sie eine erste Gruppe von Ultraschallbildern aus einer Vielzahl von Einzelbildern, die der Vielzahl von tomographischen Ebenen der Blase des Patienten entlang einer seitlichen Richtung entsprechen, und eine zweite Gruppe von Ultraschallbildern aus einer Vielzahl von Einzelbildern, die der Vielzahl von tomographischen Ebenen der Blase des Patienten entlang einer Längsrichtung entsprechen, erfasst,
die Blasenextraktionseinheit (10) so konfiguriert ist, dass sie den Blasenbereich aus jeder der ersten Gruppe der Ultraschallbilder aus der Vielzahl von Bildern und der zweiten Gruppe der Ultraschallbilder aus der Vielzahl von Bildern extrahiert,
die Merkmalswert-Berechnungseinheit (11) so konfiguriert ist, dass sie den Merkmalswert aus jedem der ersten Gruppe der Ultraschallbilder der Vielzahl von Rahmen und der zweiten Gruppe der Ultraschallbilder der Vielzahl von Rahmen berechnet, und
die Abtasterfolgs/Fehlschlag-Bestimmungseinheit (12) konfiguriert ist, um zu bestimmen, ob das Abtasten der Blase des Subjekts mit dem Ultraschallstrahl für jede der ersten Gruppe der Ultraschallbilder der Vielzahl von Bildern und der zweiten Gruppe der Ultraschallbilder der Vielzahl von Bildern erfolgreich ist oder nicht.

19. Ultraschall-Diagnosegerät nach Anspruch 18, ferner umfassend:
eine Blasenvolumen-Berechnungseinheit (15), die so konfiguriert ist, dass sie ein Volumen der Blase auf der Grundlage eines maximalen Durchmessers des Blasenbereichs, der aus der ersten Gruppe der Ultraschallbilder der Vielzahl von Rahmen in der seitlichen Richtung gemessen wird, eines maximalen Durchmessers des Blasenbereichs, der aus der zweiten Gruppe der Ultraschallbilder der Vielzahl von Rahmen in der Längsrichtung gemessen wird, und eines maximalen Durchmessers des Blasenbereichs, der aus der ersten Gruppe der Ultraschallbilder der Vielzahl von Rahmen oder der zweiten Gruppe der Ultraschallbilder der Vielzahl von Rahmen in einer Tiefenrichtung gemessen wird, berechnet.

20. Verfahren zur Steuerung eines Ultraschall-Diagnosegeräts, wobei das Verfahren umfasst:
Erfassen von Ultraschallbildern einer Vielzahl von Einzelbildern, die einer Vielzahl von verschiedenen tomographischen Ebenen in einem Subjekt bzw. einer Person entsprechen, unter Verwendung einer Ultraschallsonde, die mit der Person in Kontakt gebracht wird und die Person mit einem Ultraschallstrahl abtastet;
Extrahieren einer Blasenregion aus jedem der Ultraschallbilder aus der Vielzahl der Bilder;
**dadurch gekennzeichnet, dass** das Verfahren ferner eine Berechnung eines Merkmalswerts in Bezug auf den Blasenbereich, der in jedem der Ultraschallbilder der mehreren Bilder extrahiert wurde; und
ein Analysieren der Änderung des berechneten Merkmalswerts zwischen in zeitlicher Reihe fortlaufenden Bildern und Bestimmen, ob das Abtasten einer Blase des Subjekts mit dem Ultraschallstrahl erfolgreich ist oder nicht, basierend auf einem Analyseergebnis, umfasst.

## Revendications

1. Appareil de diagnostic par ultrasons (1) comprenant :
une sonde à ultrasons (2) configurée pour être mise en contact avec un sujet et balayer le sujet avec un faisceau d'ultrasons ;
une unité d'acquisition d'images (8) configurée pour acquérir des images ultrasonores d'une pluralité de trames correspondant à une pluralité de plans tomographiques différents dans le sujet en utilisant la sonde ultrasonore ;
une mémoire d'images (9) configurée pour conserver les images ultrasonores de la pluralité de trames acquises par l'unité d'acquisition d'images ;
une unité d'extraction de vessie (10) configurée pour extraire une région de vessie de chacune des images ultrasonores de la pluralité de trames ;
**caractérisé en ce que** l'appareil de diagnostic par ultrasons comprend en outre une unité de calcul de valeur de caractéristique (11) configurée pour calculer une valeur de caractéristique concernant la région de la vessie extraite par l'unité d'extraction de la vessie dans chacune des images ultrasonores de la pluralité de trames ; et
une unité de détermination de succès/échec de balayage (12) configurée pour analyser le changement de la valeur de caractéristique calculée par l'unité de calcul de valeur de caractéristique entre des images continues dans une série temporelle et configurée pour déterminer si le balayage d'une vessie du sujet avec le faisceau ultrasonore est réussi ou non sur la base d'un résultat d'analyse.

2. Appareil de diagnostic par ultrasons selon la revendication 1,
dans lequel les images ultrasonores de la pluralité d'images sont des images ultrasonores qui sont acquises par l'unité d'acquisition d'images tout en changeant un angle d'inclinaison de la sonde à ultrasons sur une plage d'angles donnée par rapport au sujet alors qu'une position de contact de la sonde à ultrasons avec le sujet est fixe.

3. Appareil de diagnostic par ultrasons selon la revendication 2, comprenant en outre :
une unité de mesure de diamètre maximum (13) configurée pour mesurer un diamètre maximum de la région de la vessie en utilisant les images ultrasonores de la pluralité de trames dans un cas où l'unité de détermination de réussite/échec de balayage détermine que le balayage avec le faisceau ultrasonore est réussi.

4. Appareil de diagnostic par ultrasons selon la revendication 2,
dans lequel les images ultrasonores de la pluralité de trames sont des images ultrasonores qui sont acquises par l'unité d'acquisition d'images dans un cas où une pluralité de fois de balayage de la vessie du sujet avec le faisceau ultrasonore sont effectuées.

5. Appareil de diagnostic par ultrasons selon la revendication 4,
dans lequel l'unité de détermination de succès/échec de balayage (12) est configurée pour déterminer si oui ou non chacune de la pluralité de fois de balayage avec le faisceau ultrasonore est réussie.

6. Appareil de diagnostic par ultrasons selon la revendication 5, comprenant en outre :
une unité de mesure de diamètre maximum (13) configurée pour mesurer un diamètre maximum de la région de la vessie en utilisant seulement des images ultrasonores d'une pluralité de trames acquises par un balayage avec le faisceau ultrasonore déterminé comme étant réussi par l'unité de détermination de réussite/échec de balayage parmi la pluralité de temps de balayage avec le faisceau ultrasonore.

7. Appareil de diagnostic par ultrasons selon l'une quelconque des revendications 2 à 6,
dans lequel la valeur caractéristique est une surface ou un diamètre de la région de la vessie extraite par l'unité d'extraction de la vessie dans l'image ultrasonore.

8. Appareil de diagnostic par ultrasons selon la revendication 7,
dans lequel l'unité de détermination de réussite/échec de balayage (12) est configurée pour déterminer si le balayage de la vessie du sujet avec le faisceau ultrasonore est réussi ou non en analysant la continuité de changement de la valeur de caractéristique entre des images continues dans une série temporelle.

9. Appareil de diagnostic par ultrasons selon la revendication 8,
dans lequel l'unité de détermination de succès/échec de balayage (12) est configurée pour déterminer que le balayage avec le faisceau d'ultrasons est réussi dans un cas où une valeur de différence des valeurs de caractéristiques entre des trames quelconques continues en série temporelle dans les images d'ultrasons de la pluralité de trames est inférieure à une valeur de seuil donnée, et est configurée pour déterminer que le balayage avec le faisceau d'ultrasons échoue dans un cas où une valeur de différence des valeurs de caractéristiques dans au moins une trame continue en série temporelle dans les images d'ultrasons de la pluralité de trames est égale ou supérieure à la valeur de seuil.

10. Appareil de diagnostic par ultrasons selon la revendication 8,
dans lequel les images ultrasonores de la pluralité de trames sont des images ultrasonores qui sont acquises par l'unité d'acquisition d'images tout en changeant l'angle d'inclinaison de la sonde à ultrasons à une vitesse constante alors que la position de contact de la sonde à ultrasons avec le sujet est fixe, et
l'unité de détermination de succès/échec de balayage (12) est configurée pour déterminer que le balayage avec le faisceau d'ultrasons est réussi dans un cas où une valeur de différence des valeurs de caractéristiques entre des trames quelconques continues dans une série temporelle dans les images d'ultrasons de la pluralité de trames est inférieure à une valeur de seuil donnée, et la symétrie d'une distribution des valeurs de caractéristiques par rapport à un axe temporel est reconnue, et est configuré pour déterminer que le balayage avec le faisceau d'ultrasons échoue dans un cas où une valeur de différence des valeurs de caractéristiques dans au moins une trame continue dans une série temporelle dans les images ultrasonores de la pluralité de trames est égale ou supérieure à la valeur de seuil ou dans un cas où la symétrie de la distribution des valeurs de caractéristiques par rapport à l'axe temporel n'est pas reconnue.

11. Appareil de diagnostic par ultrasons selon la revendication 10,
dans lequel l'unité de détermination de succès/échec de balayage (12) est configurée pour déterminer que la symétrie de la distribution des valeurs caractéristiques par rapport à l'axe de temps est reconnue dans un cas où une position où la valeur caractéristique est maximisée est positionnée dans un point central d'une paire de positions où la valeur caractéristique est minimisée, sur l'axe du temps, et est configuré pour déterminer que la symétrie de la distribution des valeurs caractéristiques par rapport à l'axe du temps est reconnue dans un cas où la position où la valeur caractéristique est maximisée s'écarte du point central de la paire de positions où la valeur caractéristique est minimisée, sur l'axe du temps.

12. Appareil de diagnostic par ultrasons selon la revendication 8, comprenant en outre :
un capteur d'angle d'inclinaison configuré pour mesurer l'angle d'inclinaison de la sonde à ultrasons,
dans lequel l'unité de détermination de succès/échec de balayage (12) est configurée pour déterminer que le balayage avec le faisceau d'ultrasons est réussi dans un cas où une valeur de différence des valeurs de caractéristiques entre toutes les trames continues dans la série temporelle dans les images d'ultrasons de la pluralité de trames est inférieure à une valeur de seuil donnée, et une position où la valeur de caractéristique indique une valeur extrême est reconnue comme coïncidant avec une position où l'angle d'inclinaison mesuré par le capteur d'angle d'inclinaison indique une valeur extrême, sur un axe de temps, et est configuré pour déterminer que le balayage avec le faisceau d'ultrasons échoue dans un cas où une valeur de différence des valeurs de caractéristiques dans au moins une image continue en série temporelle est égale ou supérieure à la valeur de seuil ou la position où la valeur de caractéristique par rapport à l'axe de temps indique la valeur extrême n'est pas reconnue comme coïncidant avec la position où l'angle d'inclinaison mesuré par le capteur d'angle d'inclinaison indique la valeur extrême.

13. Appareil de diagnostic par ultrasons selon la revendication 12, comprenant en outre :
une unité d'estimation de distance cible configurée pour estimer une distance entre un centre de la vessie du sujet et la position de contact de la sonde à ultrasons avec le sujet dans une direction le long d'une surface corporelle du sujet sur la base d'une image à ultrasons d'une trame représentant un plan tomographique passant par le centre de la vessie du sujet parmi les images à ultrasons de la pluralité de trames acquises par l'unité d'acquisition d'images et l'angle d'inclinaison de la sonde à ultrasons mesuré par le capteur d'angle d'inclinaison ; et
une unité de guidage de mouvement de sonde configurée pour guider un utilisateur afin de positionner la sonde à ultrasons directement au-dessus du centre de la vessie du sujet en déplaçant la sonde à ultrasons le long de la surface du corps du sujet par la distance estimée par l'unité d'estimation de distance cible.

14. Appareil de diagnostic par ultrasons selon l'une quelconque des revendications 2 à 6,
dans lequel la valeur caractéristique est une position de la région de la vessie extraite par l'unité d'extraction de la vessie dans l'image ultrasonore.

15. Appareil de diagnostic par ultrasons selon la revendication 14,
dans lequel la valeur caractéristique est une position d'un centre de gravité de la région de la vessie extraite par l'unité d'extraction de la vessie dans l'image ultrasonore, et
l'unité de détermination de succès/échec de balayage (12) est configurée pour déterminer que le balayage avec le faisceau d'ultrasons est réussi dans un cas où une distance entre les centres de gravité des régions de la vessie dans les trames continues dans la série temporelle est inférieure à une valeur de seuil donnée, et est configurée pour déterminer que le balayage avec le faisceau d'ultrasons échoue dans un cas où la distance entre les centres de gravité des régions de la vessie dans les trames continues dans la série temporelle est égale ou supérieure à la valeur de seuil donnée.

16. Appareil de diagnostic par ultrasons selon la revendication 14,
dans lequel l'unité de détermination de succès/échec de balayage (12) est configurée pour déterminer que le balayage avec le faisceau d'ultrasons est réussi dans un cas où un rapport d'une zone d'une région où les régions de la vessie se chevauchent les unes les autres sur une zone d'une région occupée par au moins une des régions de la vessie dans les trames continues dans la série temporelle est égal ou supérieur à une valeur de seuil donnée, et est configuré pour déterminer que le balayage du faisceau d'ultrasons échoue dans un cas où le rapport de l'aire de la région où les régions de la vessie se chevauchent sur l'aire de la région occupée par au moins une des régions de la vessie dans les images continues dans la série temporelle est inférieur à la valeur de seuil donnée.

17. Appareil de diagnostic par ultrasons selon l'une quelconque des revendications 1 à 16, comprenant en outre :
une unité de notification (14) qui, dans un cas où l'unité de détermination de réussite/échec de balayage détermine que le balayage du faisceau d'ultrasons échoue, est configurée pour notifier à un utilisateur que le balayage du faisceau d'ultrasons échoue.

18. Appareil de diagnostic par ultrasons selon l'une quelconque des revendications 1 à 17,
dans lequel l'unité d'acquisition d'images (8) est configurée pour acquérir un premier groupe d'images ultrasonores d'une pluralité de cadres correspondant à la pluralité de plans tomographiques de la vessie du sujet le long d'une direction latérale et un second groupe d'images ultrasonores d'une pluralité de cadres correspondant à la pluralité de plans tomographiques de la vessie du sujet le long d'une direction longitudinale,
l'unité d'extraction de vessie (10) est configurée pour extraire la région de vessie de chacun du premier groupe d'images ultrasonores de la pluralité de trames et du second groupe d'images ultrasonores de la pluralité de trames,
l'unité de calcul de valeur de caractéristique (11) est configurée pour calculer la valeur de caractéristique à partir de chacun du premier groupe d'images ultrasonores de la pluralité de trames et du second groupe d'images ultrasonores de la pluralité de trames, et
l'unité de détermination de succès/échec de balayage (12) est configurée pour déterminer si le balayage de la vessie du sujet avec le faisceau ultrasonore est réussi ou non pour chacun du premier groupe d'images ultrasonores de la pluralité de trames et du second groupe d'images ultrasonores de la pluralité de trames.

19. Appareil de diagnostic par ultrasons selon la revendication 18, comprenant en outre :
une unité de calcul du volume de la vessie (15) qui est configurée pour calculer un volume de la vessie sur la base d'un diamètre maximal de la région de la vessie mesuré à partir du premier groupe d'images ultrasonores de la pluralité de cadres dans la direction latérale, d'un diamètre maximal de la région de la vessie mesuré à partir du second groupe d'images ultrasonores de la pluralité de cadres dans la direction longitudinale, et d'un diamètre maximal de la région de la vessie mesuré à partir du premier groupe d'images ultrasonores de la pluralité de cadres ou du second groupe d'images ultrasonores de la pluralité de cadres dans une direction de profondeur.

20. Procédé de commande d'un appareil de diagnostic par ultrasons, le procédé comprenant :
l'acquisition d'images ultrasonores d'une pluralité de cadres correspondant à une pluralité de plans tomographiques différents dans un sujet en utilisant une sonde ultrasonore qui est amenée en contact avec le sujet et balaie le sujet avec un faisceau ultrasonore ;
conserver les images ultrasonores acquises de la pluralité de trames ;
l'extraction d'une région de la vessie à partir de chacune des images ultrasonores de la pluralité de trames ;
**caractérisé en ce que** le procédé comprend en outre le calcul d'une valeur de caractéristique concernant la région de la vessie extraite dans chacune des images à ultrasons de la pluralité de trames ; et
analyser le changement de la valeur de caractéristique calculée entre les images continues dans la série temporelle et déterminer si le balayage d'une vessie du sujet avec le faisceau ultrasonore est réussi ou non sur la base d'un résultat d'analyse.
